(19) Europäisches Patentamt
European Patent Office
Office européen des brevets

(11) **EP 4 249 016 A1**

(12) **EUROPEAN PATENT APPLICATION**

(43) Date of publication:
**27.09.2023   Bulletin 2023/39**

(51) International Patent Classification (IPC):
**A61M 5/20** $^{(2006.01)}$    **A61M 5/315** $^{(2006.01)}$
**A61M 5/24** $^{(2006.01)}$

(21) Application number: **21315262.2**

(22) Date of filing: **24.03.2022**

(52) Cooperative Patent Classification (CPC):
**A61M 5/24;** A61M 2205/3327

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **SANOFI**
**75008 Paris (FR)**

(72) Inventor: **The designation of the inventor has not
yet been filed**

(74) Representative: **Sanofi Aventis Deutschland
GmbH
Industriepark Höchst
Geb. K703
65926 Frankfurt am Main (DE)**

(54) **ACTUATING UNIT FOR A DRUG DELIVERY DEVICE AND RELATED ITEMS**

(57)     Disclosed is an actuating unit (301) for a drug delivery device (100), comprising:
- A support structure (300) extending along a longitudinal axis (A) between a distal (D) portion of the support structure (300) and a proximal (P) portion of the support structure (300), and
- An interface unit (IF1, IF2) arranged within the support structure (300) and comprising a coupling portion (340) and an actuating portion (332),
wherein the coupling portion (340) is configured to interact with a rotating member comprising a plurality of teeth (404a) and rotating around a rotation axis (A) that is parallel or coaxial to the longitudinal axis (A),
wherein the actuating portion (332) is configured to interact with an electrical component (Sw), wherein the interface unit (IF1, IF2) is configured to interact with the plurality of teeth (404a) of the rotating member and to convert a rotational movement (R) of the rotating member to an axial displacement (Arr7Bp) of the coupling portion (340) in a first direction relative to the longitudinal axis (A), e.g. from a first axial position to a second axial position, and to a radial displacement of at least a part of the coupling portion (340) relative to the longitudinal axis, and wherein the axial displacement and/or the radial displacement of the coupling portion (340) displaces the actuating portion (332).

Fig. 3

**Description**

The disclosure relates to an actuating unit for a drug delivery device or of a drug delivery device. Furthermore, the disclosure relates to a rotating member interacting with the actuating unit and to a drug delivery device comprising the actuating unit and/or the rotating member.

The drug delivery device may be an autoinjector or a manually or semi-automatically operated device like a user actuated pen injector with our without a dial extension. An energy storing element may be used in autoinjectors as well as in semi-automatically operated (spring driven) devices in order to deliver the driving force for the injection operation. The energy storing element may be biased in the factory or by the user prior to use. The drug may comprise insulin or GLP-1 (Glucagon-Like Peptide). However, other drugs may also be injected. Furthermore, other medical devices may also profit from the disclosure, e.g. injectors, spraying devices or inhalation devices.

Usually, drug delivery devices may comprise a lot of mechanical components. However, there may be the desire to use electronic components together with mechanical components. Power management of the electronic circuit may be important in order to have elongated use of a battery or of an accumulator for storing electrical energy.

It is an object of the disclosure to provide an actuating unit for a drug delivery device. The actuating unit should be preferably easily and/or comfortably to use and/or comprise as few parts as possible. Furthermore, preferably easy assembling of the actuating unit itself and/or easy assembling of the actuating unit to the drug delivery device should be possible. Moreover, preferably the actuating unit shall be small in size and/or should allow the usage of drug delivery devices that are known without modification or with only slight modifications. Furthermore, a corresponding rotating member, a drug delivery device and a method shall be provided.

This object is solved by the actuating unit according to claim 1. Further embodiments are given in the dependent claims. The subject matter of the other independent claims solve the object too.

According to an embodiment, an actuating unit for a drug delivery device is provided, comprising:

- A support structure extending along a longitudinal axis between a distal portion of the support structure and a proximal portion of the support structure, and/or
- An interface unit arranged within the support structure and comprising a coupling portion and an actuating portion.

According to an embodiment, the coupling portion may be configured to interact with a rotating member comprising a plurality of teeth and rotating around a rotation axis parallel or coaxial to the longitudinal axis. The rotating member may be or may be part of a dial sleeve and/or a number sleeve. However, the rotating member may be another rotating part or may be coupled to another rotating part of a drug delivery device.

According to an embodiment, the actuating portion may be configured to interact with an electrical component, e.g. with an electrical switch and/or with an electrical or electronic sensor unit.

According to an embodiment, the interface unit may be configured to interact with a plurality of teeth of the rotating member and to convert a rotational movement of the rotating member to an axial displacement of the coupling portion, e.g. in a first axial direction, relative to the longitudinal axis from, e.g. a first axial position to a second axial position, and to (in) a radial displacement of at least a part of the coupling portion relative to the longitudinal axis, e.g. from a first radial position to a second radial position.

According to an embodiment, the axial displacement and/or the radial displacement of the coupling portion may also displace the actuating portion. Thus, a switch may be switched on or off. Alternatively, a sensor unit may detect the displacement of the actuating portion and may generate a signal that is used to switch on or to switch off other electronic units of the drug delivery device. Thus, the device may be switched from a low energy state to a high energy state in which the drug delivery device uses more electrical energy compared to the low energy state. This means that the electronic unit of the drug delivery device may be woken up.

According to an embodiment, the direction and/or amount of the radial displacement may be appropriate to hold the coupling portion with a holding structure, e.g. arranged on the rotating member. Re-engagement of the coupling portion with the teeth of the rotating member during dispense of a dose may be prevented thereby. However, there might be some concepts which wouldn't need this 'holding structure' part of the device to work and to have the technical effects mentioned in this application or other technical effects.

According to a further embodiment the holding structure(s) may be arranged on the teeth of the rotating member. Alternatively the holding structure(s) may be arranged inwards or outwards of the teeth, e.g. with a gap between the holding structure(s) and the teeth. The holding structure(s) may be arranged on the rotating member or on a part that is close to the rotating member, e.g. a housing part.

The technical effect of the embodiment(s) may be that the rotating member may rotate without further breaking influence of the teeth after the first axial displacement and/or radial displacement, e.g. only some rotation energy is used for sliding on holding structure. Low losses by friction are possible therefore. Thus, a driving force for injection of a dose of the drug may be used mainly for the injection, i.e. the force of a drive spring and/or the force generated by a user during injection. Thus, it may be possible to wake up an electronic circuit of the drug delivery device as short as possible after the beginning of the rotation of the rotating member. Thus, energy for a further sensor unit that detects the rotation of the rotating member or of a rotating part coupled thereto may be used efficient.

The supporting structure may be a cylinder or may be cylindrical. The diameter of supporting structure may be in the range of 1 cm (centimeter) to 2 cm. The length of the supporting structure may be in the range of 1 cm to 2 cm, too. The amount of radial displacement may be in the range of 0.25 mm (millimeter) to 0.8 mm or may be at least 0.25 mm. The amount of axial displacement may be in the range 0.25 mm to 0.8 mm or may be at least 0.25 mm or at least 0.3 mm or at least 0.5 mm allowing mass production of the parts by cost effective injection molding and/or using greater tolerances that allow a high yield and/or are sufficient to guarantee safe function of the actuating unit, especially during interaction with the rotating member.

A ratio between the amount of the axial displacement and the amount of the radial displacement may be in the range of 3 to 1 or in the range from 1.5 to 1.

Axial displacement may be in the proximal direction, e.g. away from a needle or nozzle of the drug delivery device.

The radial displacement may be from a first radial position to a second radial position that is radial outwards of the first radial position relative to the longitudinal axis or relative to the rotation axis. However, in other examples the second radial position may be radially inwards relative to the first radial position.

A radial outward displacement may be realized using a retaining element of the coupling portion extending cross to the longitudinal axis and/or using a radially extending protrusion on the supporting structure or on the mechanical actuating element.

The coupling portion and the actuating portion may be arranged on the same element, e.g. on a coupling feature. Alternatively, they may be arranged on different elements that are coupled mechanically together in an appropriate way.

According to an embodiment, the interface unit may comprise a retaining element that retains the coupling portion on a retaining portion of the retaining element. The retaining element may be attached to the supporting structure with an attachment portion of the retaining element. The retaining element may be resilient (elastically deformable) and/or may be attached pivoting or pivotable to the supporting structure thereby allowing the distal displacement and the radial displacement of the coupling portion. An elongated retaining element (arm) or ring, or other structure may be used. A ring or curved arms may allow the arrangement of other parts within a central opening of the rotation member.

There may be no pivoting point of the retaining element on an intermediate portion or on a portion attached to an intermediate portion of the retaining element. Thus, only one attachment point or several attachment points at a peripheral portion may be used. The length of a pivoting part measured from the pivoting point may be increased thereby. Larger length may result in larger axial displacement and/or radial displacement of the coupling portion.

According to an embodiment, the coupling portion may be configured to interact with an interacting portion on a respective one of the teeth of the rotating member of the drug delivery device such that the rotating member acts a force on the coupling portion, e.g. a circumferential or angular force. The rotating member or another part may comprise at least one holding structure arranged radially outwards or radially inwards of the interaction portion of the rotating member. The holding structure may comprise at least one proximal directed surface configured to allow sliding of the distal end of the coupling portion thereon during further rotation of the rotating member.

Thus, e.g. the radially outwards displacement of the distal end of the mechanical actuating element may be used or is used to prevent that the coupling portion moves axially back, e.g. into a second pocket, e.g. an inner pocket, between two teeth of the rotating member. A ramped feature may be used on coupling portion and/or on the supporting structure in order to ease axial movement due to a circumferential force.

According to an embodiment, the actuating unit may comprise a coupling feature. The coupling portion may be comprised or may be a part of the coupling feature. The coupling feature may comprise at least one curved surface configured to interact with a guiding feature arranged on the supporting structure when a circumferential force is applied by the rotating member to the coupling portion thereby displacing the coupling feature and/or the coupling portion axially. Thus, the conversion of a rotational movement into an axial movement may be easier. A ramped guiding feature may be used, see ramped feature mentioned in the description of the figures. Alternatively, a curved surface may only be used on the guiding feature but not on the coupling feature as is describe below in more detail.

According to an embodiment, the guiding feature may comprise at least one curved surface opposite to the curved surface of the coupling feature. Both curved surfaces may be formed complementary to each other, e.g. at least within a respective portion thereby promoting the axial displacement of the coupling feature.

Both curved surfaces may be completely engaged at the beginning of rotation of the rotation member. Thus, at least one concave edge of one curve may be adjacent to a convex edge of the other curve and/or vice versa. Furthermore, a concave portion of one curve may be adjacent to a convex portion of the other curve. Radii of curvature may be the same at portions which are opposite to one another in both curves.

According to an embodiment, the coupling portion may comprise at least two distal portions and a recessed portion arranged between the at least two distal portions, e.g. the coupling portion may be comb-like comprising at least two prongs or claws, e.g. a bifurcation. The recessed portion may be recessed proximally relative to the at least two distal portions. Thus, due to this design, a number of positions of the rotating member after dose dialing may be twice the number of teeth on the rotating member. Thus, coarser structures may be used on rotating member and/or on the coupling member compared to the case in which both numbers are identical. This may reduce costs and/or raise yield in the production of the actuating member and/or rotating member, e.g. using injection molding. It may also improve the robustness and/or performance of the rotating member or the components that it interfaces with.

According to an embodiment, a first distal portion of the at least two distal portions may comprise a first side face of the recessed portion and a second distal portion of the at least two distal portions may comprise a second side face of the recessed portion. The second side face may be longer than the first side face. This may be due to the usage of a ramped surface at a proximal side of the recessed portion. The first side face may be at least 10 percent shorter or at least 20 percent shorter relative to the length of the second side face.

A third face of the recess may be arranged between the proximal end of the first side face and the proximal end of the second face. Thus, the recess may be unsymmetrical relative to an axis located in the middle between the two side faces and/or parallel to the two side faces.

The technical effect may be, that there is better engagement of the coupling portion (coupling feature) with the teeth of the rotating member. Better engagement may allow the production of parts having greater tolerances.

According to a further aspect, a rotating member is disclosed, comprising a ring member. According to an embodiment, a plurality of teeth may be arranged circumferentially on the ring member around an axis of rotation of the ring member.

According to an embodiment, each tooth may comprise an inner portion and an outer portion arranged radially outwards for the respective inner portion.

According to an embodiment, the inner portions may comprise first teeth comprising at least one ramped face that faces in a proximal direction relative to the axis of rotation and in circumferential direction of the ring member. Such teeth may be known as crown teeth.

According to an embodiment, the outer portions may comprise second teeth of a different orientation and/or type compared to the first teeth. Thus, the rotating member may comprise at least two or at least three different types of sub-teeth arranged on each tooth, e.g. a first type of teeth on a most radial inward position, a second type of teeth at a middle position but radially outwards of the first type and/or a third type of teeth at a radial outwards position relative to the middle position. Multifunctional use of the teeth may be possible therefore.

According to a further embodiment, the second teeth may comprise a flat face that is arranged in a plane perpendicular (e.g. in the range from 85 to 95 degree) to the axis of rotation of the rotating member or of the ring member. The second teeth may have an angular width that is greater than an angular width of the first teeth. Thus, the second teeth may form the holding structure mentioned above, e.g. preventing that coupling portion "falls back" into the pockets or spaces between the teeth, e.g. the teeth of the first type. This kind of holding structure may simplify production, e.g. compared to a continuous ring portion instead of the separate holding portions. However, such a ring structure may have other advantages. Moreover such a ring structure may be provided separate from the teeth of the rotating member, e.g. arranged on the rotating member or on another appropriate part of the actuating unit or on the drug delivery device.

According to a further embodiment, a most proximal portion of the first teeth or a ramp starting on this most proximal portion of the first teeth may extend up to the plane and/or may have the same axial position as the adjacent flat face of the second teeth. This, may ease the radial movement/ displacement e.g. at the end of the axial displacement because no step or only a small step has to be crossed.

Alternatively, a radially directed ramp feature on the teeth and/or on the coupling portion may be used allowing to use of different axial positions for the most proximal portion/top faces (proximal facing faces) of the first teeth and of the second teeth.

According to a further embodiment, each first teeth may have at least one face that faces in the same direction as a face of the second teeth, e.g. a back face. Thus, production, e.g. injection molding may be easier.

According to a further embodiment, the teeth may have constant distances to adjacent teeth, i.e. a constant pitch.

The outer portion may be adjacent to the inner portion, i.e. no other portion may be arranged therein between. Alternatively, other portions may be arranged between both portions.

According to an embodiment, the outer portions may comprise respectively an inner sub-portion and an outer sub-portion arranged radially outwards of the inner sub-portion. The inner sub-portion may be used as the holding structure preventing re-engagement during dose dispense. The inner sub-portion may have an angular width that is larger than the angular width of the outer sub-portion. It may be possible to connect the inner sub portion in a circumferential direction in order to reduce friction between the coupling portion and the rotating member further.

The outer sub-portion(s) may have further coupling functions and/or may be used as rotation encoding element in order to measure the dose dispensed and/or dialed. Thus, proximal faces or radial facing faces or other appropriate faces of the outer sub-portion(s) may be used as reflecting faces that reflect electromagnetic radiation, e.g. light or infrared, to a sensor that is used for rotation detection.

Respective inner sub-portions and outer sub-portions may have at least one face or at least two faces or at least three faces facing in the same direction, e.g. "top" or proximal face and back face if compared to direction of rotation of the ring member, and/or bottom surface. This may simplify design and/or production.

The respective outer sub-portion may be adjacent to the respective inner sub-portion, i.e. no other portion may be arranged therein between. Alternatively, other portions may be arranged between both portions.

According to a further embodiment, the rotating member may be made of plastic, e.g. using injection molding. Thus, the teeth of the rotating member may be integrally formed with the ring member. Cost effective production may be possible by producing tens or hundreds of parts with one injection into one molding tool.

According to a further embodiment, the rotating member may be part of a number sleeve of a drug delivery device or may be a part connected to a number sleeve, e.g. a drive sleeve. The connection may a snap connection. Other types of connection may also be used, e.g. using adhesives.

A further aspect relates to a drug delivery device, comprising:

- A housing, preferably an elongated housing, extending along a longitudinal axis, and/or
- A dose setting unit and/or a drug delivery unit arranged within the housing, and/or
- An actuating unit as mentioned above, and/or
- A rotating member as mentioned above.

Thus, the same technical effects as mentioned above may also be valid for the drug delivery device.

According to an embodiment, the actuating unit may be arranged on or within a proximal part of the housing. The actuating unit may comprise an electric component that may be actuated by the actuating portion of the actuating unit, e.g. an electric switch (micro switch, etc.) or a sensor, e.g. a magnetic sensor.

According to a further embodiment, the rotating member may be part of or may be mechanically coupled to the dose setting unit and/or to the dose delivery unit. The rotating member may preferably be arranged such that it interacts with the actuating unit.

The dose setting unit may comprise a number sleeve or dial sleeve, e.g. indicating the amount of a selected or dialed dose and/or a drive sleeve that may e.g. be used to set dose.

The drug delivery unit may comprise a plunger rod and/or a drive sleeve. The drive sleeve may interact with the plunger rod during drug delivery. Alternatively or additionally, a drive spring may be used, e.g. a torsion spring or a compression spring or a tension spring. Advanced embodiments may use an electrical motor for drug delivery, e.g. drug injection.

According to a further embodiment, the drug delivery device may be autoinjector, comprising e.g. a drive spring and/or distal needle shroud for starting automatic injection when pressed against the skin of a patient. Alternatively, a manually driven device may be used. Furthermore, advanced drug delivery devices that may be used may comprise an electrical motor used for dose delivery.

The actuating unit may form a module, preferably an electronic module/actuating unit. The module may be configured to be used with at least two drug delivery devices or with at least three drug delivery devices. Thus, multiuse of the valuable electronic module may be possible. However, alternatively the actuating unit/electronic unit may be integral part of the drug delivery device, e.g. no change between two drug delivery devices may be possible without destroying the actuating unit and/or the first drug delivery device. This may prevent manipulation of the electronic unit and/or of the drug delivery device.

According to a further embodiment, the electronic unit/module may comprise further electronic units, e.g. connected to the electrical component:

- A processing unit, and/or
- A memory unit, and/or
- An electronic power management unit, and/or
- A communication unit, e.g. near field communication, Bluetooth, ZigBee, etc., and/or
- At least one sensor unit configured to detect or measure the amount of delivered and/or set dose, and/or
- A detection or measuring unit, e.g. comprising the at least one sensor unit.

The sensor unit may comprise at least one optical sensor. However, other sensor principles may be used as well, magnetic, capacitive, inductive, etc., preferably contactless sensors. However, sensor making mechanical contact may be used as well.

According to an embodiment, the dose setting unit and/or the dose delivery unit may be configured to position the rotating member in one of a number of positions. The number of teeth, e.g. of inner teeth, of the rotating member may be equal to one half of the number of positions. This may have the technical effect that exact positioning of the coupling portion relative to the greater number of positions may still be possible, e.g. by using a pronged or comb-like coupling portion. Larger teeth may be used by reducing the number of teeth relative to a given diameter of the rotating member. Yield of the production may thus be higher and/or tolerances may be chosen larger.

According to an embodiment, at least one, at least two, at least three or all of the following equations may valid:

-

$$W1a > W2a, (1),$$

wherein W1a may be the angular width of a recessed portion or of the recessed portion of the coupling portion, and wherein W2a may be the angular width of an inner tooth or of the inner tooth of the rotating member.

Preferably W1a may be at most 1 percent or 5 percent longer than W2a, and/or

-

$$D2b > W1b, (2),$$

wherein D2b is the angular distance between a back face of a first one of the inner tooth and the front face an adjacent inner tooth, and wherein W1b is the angular width of the coupling portion, e.g. distal width.

Preferably D2b may be at most 1 percent or 5 percent longer than W1b, and/or

-

$$W3a > W1a, (3),$$

wherein W3a is the angular width of an inner sub-portion of the outer portions, and wherein W1a is the angular width of a recessed portion or of the recessed portion of the coupling portion.

Preferably W3a may be at least 10 percent longer than W1a. An upper limit may be chosen appropriately, at e.g. most 30 percent or at most 50 percent longer, and/or

-

$$W1b > D3b, (4),$$

wherein W1b is the angular width of the coupling portion.

Preferably W1b may be at least 5 percent or at least 10 percent longer than D3b. An upper limit may be chosen appropriately, at e.g. most 30 percent or at most 50 percent longer, and wherein D3b is the angular distance between a back face of an inner sub-portion of the outer portion and a front face or a front edge of the adjacent inner sub-portion of the adjacent outer portion.

Equations (1) and (2) may make sure that the coupling portions engage the teeth of the rotating member even in cases where there are more relative positions of the rotating members after dialing and/or delivering of a dose compared to the number of teeth, e.g. inner teeth on the rotating member. Thus, the number of positions may be twice the number of e.g. inner teeth, e.g. there may be 24 positions and only 12 teeth, e.g. at the same radial position.

Equations (3) and (4) may make sure that the coupling portion does not re-engage with the inner teeth when in the coupling portion or at least a part of the coupling portion is in the radial deflected position, e.g. the coupling portion may slide on the inner sub-portions of the outer portions mentioned above. Outer sub-portions of the outer portions may be optional.

According to an embodiment, the coupling portion may be configured to interact with an interacting portion, e.g. inner tooth/teeth, on a respective one of the teeth of the rotating member of the drug delivery device such that the rotating member may act a force on the coupling portion. The rotating member may comprise at least one holding structure arranged radially outwards of the interaction portion of the rotating member. The holding structure may comprise at least one proximal directed surface configured to allow sliding of the distal end of the coupling portion thereon during further rotation of the rotating member.

According to a further embodiment, the actuating unit may comprise a coupling feature wherein the coupling portion may be comprised within the coupling feature. The coupling feature may comprise at least one curved surface configured to interact with a guiding feature arranged on the supporting structure when a circumferential force is applied to the coupling portion thereby displacing the coupling feature axially.

According to a further embodiment, the coupling feature may comprise two distal portion and a recessed portion arranged between the two distal portions.

According to an embodiment the following equation may be valid:

$$- W3a > W3b, (5),$$

wherein W3a may be the angular width of an inner sub-portion of the outer portions, and W3b may the angular width of an outer sub-portion of the outer portions.

W3a may be at least 10 percent longer compared to length W3b. An upper limit of W3a may be chosen appropriately, at e.g. most 30 percent or at most 50 percent longer compared to W3b. The outer sub-portion may have at least one further function, e.g. a coupling function to at least one further mechanical element of the drug delivery device, etc. The outer sub-portion(s) may be used as rotation encoding element in order to measure the dose dispensed and/or dialed. Alternatively, a rotation encoding element, e.g. an encoding ring may be engaged with the outer sub-portion(s). Thus, proximal faces or radial facing faces or other appropriate faces of the outer sub-portion(s) or of the additional encoder ring may be used as reflecting faces that reflect electromagnetic radiation, e.g. light or infrared, to a sensor that is used for rotation detection.

An aspect relates to a method for actuating a drug delivery device, comprising:

- Pressing an actuating unit distally thereby engaging a coupling portion and teeth of a rotating member, whereby preferably an actuating portion of the actuating unit does not actuate on an electrical component,
- Thereafter, rotating the rotating member thereby disengaging the coupling portion out of a pocket formed between adjacent teeth, whereby an actuating portion or the actuating portion of the actuating unit acts on the electrical component, and
- Thereafter, preventing the coupling portion from re-engaging pockets between the teeth, e.g. by using a holding structure arranged radially outwards or radially inwards relative to the teeth, whereby preferably the actuating portion of the actuating unit acts further on the electrical component.

The technical effect may be that the rotating member may rotate without further breaking influence of the teeth after the first and only axial displacement of one working cycle, i.e. only some rotation energy may be used for the first axial displacement and/or radial displacement and then for sliding of the coupling portion on the holding structure. Low friction losses may occur.

According to a further embodiment, there may be at least one further method step:

- Stopping pressing on the actuating unit,
- Preferably, thereafter disengaging the coupling portion from the holding structure, e.g. in order to establish the original conformation, e.g. a released conformation.

According to a further embodiment, actuating on the electric component may wake up the main electronic unit of the electronic unit/module of the drug delivery device. Thus, a second energy state with more energy consumption than a first energy state, e.g. sleep state, may be used thereby prolonging the life of a battery or accumulator and/or the time between two charging actions of an accumulator of the drug delivery device and/or of the electronic unit.

According to a further embodiment, the method may use the actuating unit and its embodiments as mentioned above. Thus, the same technical effects that have mentioned above may apply also to the method. Vice versa, the method may be used to operate the actuating unit as mentioned above. Thus, technical effects of the method may be valid for the actuating unit. The same is valid for the rotating member and/or for the drug delivery device with regard to the application of the method and vice versa.

According to a further embodiment of the method, a guiding feature, e.g. on a support structure (chassis) of the actuating unit, may be used to initiate a proximal displacement of the coupling portion. A ramp on a teeth may be used thereafter to displace the coupling portion further proximally. The following technical effect may be realized: large engagement between coupling portion and rotation member and/or comparably low rotation force necessary to initiate and perform proximal movement/displacement of coupling portion. Moderate steepness of angled or ramped face(s) on coupling portion/coupling feature and/or on the guiding feature.

According to a further embodiment, at the end of the proximal displacement, a radial displacement of the coupling portion may be performed. The radial displacement may be radially outwards or radially inwards. Radial displacement may be used to move e.g. distal face of coupling portion onto a holding structure, for instance a holding structure arranged on the rotating member, especially on the teeth of the rotating member.

According to a further embodiment, an annular holding ring (sleeve) may be used instead of the holding structures arranged on the teeth. The holding ring may rotate with rotating member and/or may surround teeth of rotating member, e.g. a circular sleeve.

A next aspect relates to a system. According to an embodiment, the system may comprise a drug delivery device and/or an electronic module. According to an embodiment, the drug delivery device may comprise a rotating member comprising a plurality of teeth. According to an embodiment, the electronic module may comprise an electrical component and/or an electrical energy source and/or an energy management unit electrically connected to the at least one electrical component. According to an embodiment, the energy management unit may be configured to switch depending on an electrical signal generated by the electrical component from a low energy state to a higher energy state that consumes more electrical energy from the electrical energy source compared to the low energy state. According to an embodiment, the electronic module may comprise an interface unit, According to an embodiment, the interface unit may comprise a coupling portion and an actuating portion configured to interact with the electrical component. According to an embodiment, the interface unit may be configured to interact with the plurality of teeth of the rotating member by the coupling portion and to convert a rotational movement of the rotating member to an axial displacement of the coupling portion in a first axial direction relative to a longitudinal axis of the drug delivery device and to a radial displacement of at least a part of the coupling portion relative to the longitudinal axis. According to an embodiment, the interface unit may be configured to transfer the axial displacement and/or the radial displacement to the actuating portion thereby initiating switching to the higher energy state by the energy management unit. According to an embodiment, the direction and amount of the radial displacement may be appropriate to hold the coupling portion by a holding structure thereby preventing re-engagement of the coupling portion and the teeth of the rotating member during dispense of a dose.

According to a further embodiment, the interface unit may be part of an actuating unit, preferably of an actuating unit as mentioned above.

During dose delivery, the rotating member, e.g. number sleeve and/or dial sleeve may rotate and ramped features of the rotating member may engage the coupling portion. This in turn may cause the flexible coupling portion and/or the flexibly arranged coupling portion to bear rotationally against a support structure, e.g. chassis, of electronic module and to deflect axially, e.g. upwards, relative to the electronic module. Thereby, the interface unit may drive a switch axially, e.g. a micro-switch. A sensor unit may be used instead of a switch. The switch or sensor unit may wake up a processor, e.g. a microcontroller, and/or may initialize a rotation sensor of a dose capturing system, e.g. LEDs (Light emitting diodes, or IR (infrared) LEDs.

According to an embodiment, the interface unit may be configured to prevent interaction of the actuating portion and of the electrical component when the coupling portion is moved in a second axial direction that is opposite to the first axial direction. Thus, there a recessed portion on the coupling portion may be used. Alternatively or additionally, the coupling portion may have geometrical dimensions, that allow engagement of the coupling portion between two adjacent teeth of the rotating member. Furthermore, other features may be used, e.g. alignment features aligning the rotation member relative to the coupling portion.

According to a further embodiment, the drug delivery device used in the system may be a drug delivery device according to any one of the aspects and embodiments mentioned above.

According to a further embodiment, the electronic module may comprise an actuating unit according to any one of the aspects and embodiments mentioned above. According to a further embodiment, the rotating member may be a rotating member according to any one of the aspects and embodiments as mentioned above. Thus, the technical effects mentioned above may be valid for the system.

According to a further embodiment, the actuating portion may be laterally arranged on interface unit or on the coupling portion and/or on a proximal location or on another appropriate location of these parts or of other appropriate parts.

According to a further embodiment that may be valid for all aspects, the electronic module may be detachable from the drug delivery device. Thus, the electronic module may be removed and/or mounted without damaging the electronic module and/or without damaging the drug delivery device. Mounting and/or detaching the electronic module may be possible without using a separate tool. Thus, reuse of valuable part may be possible.

Alternatively, the electronic module may be an integral part of the system. Thus, the electronic module may be removed only by damaging the electronic module and/or damaging the drug delivery device. Manipulations of the electronic module and/or of the drug delivery device may be hampered in this way.

The making and using of the presently preferred embodiments are discussed in detail below. It should be appreciated, however, that the present disclosure provides many applicable concepts that can be embodied in a wide variety of specific contexts. The specific embodiments discussed are merely illustrative of specific ways to make and use the disclosed concepts, and do not limit the scope of the claims.

Moreover, same reference numerals refer to same technical features if not stated otherwise. As far as "may" is used in this application it means the possibility of doing so as well as the actual technical implementation. The present concepts of the present disclosure will be described with respect to preferred embodiments below in a more specific context namely drug delivery devices, especially drug delivery devices for humans or animals. The disclosed concepts may also be applied, however, to other situations and/or arrangements as well.

The foregoing has outlined rather broadly the features and technical advantages of embodiments of the present disclosure. Additional features and advantages of embodiments of the present disclosure will be described hereinafter, e.g. of the subject-matter of dependent claims. It should be appreciated by those skilled in the art that the conception and specific embodiments disclosed may be readily utilized as a basis for modifying or designing other structures or processes for realizing concepts which have the same or similar purposes as the concepts specifically discussed herein. It should also be recognized by those skilled in the art that equivalent constructions do not depart from the spirit and scope of the disclosure, such as defined in the appended claims.

For a more complete understanding of the presently disclosed concepts and the advantages thereof, reference is now made to the following description in conjunction with the accompanying drawings. The drawings are not drawn to scale. In the drawings the following is shown in:

| | |
|---|---|
| Figure 1 | a drug delivery device, |
| Figure 2 | an electronic unit, |
| Figure 3 | a cross section of a button inner (e.g. chassis), showing a coupling feature on a flexible arm or ring, |
| Figure 4 | ramp features on teeth of a number sleeve, |
| Figures 5A and 5B | the coupling feature as the button travels distally, straddling a single tooth, |
| Figures 6A and 6B | the coupling feature as the button travels distally, between two teeth of the number sleeve, |
| Figure 7A | a first configuration of a ring member, a coupling feature and a ramped feature, |
| Figure 7B to 7E | a second configuration to a fifth configuration of the ring member, the coupling feature and the ramped feature, |
| Figure 8 | the coupling feature in a deflected state, e.g. deflected upwards and radially outwards, |
| Figures 9A and 9B | the coupling feature configured to bridge gaps between teeth of the number sleeve, and |
| Figure 10 | a position of the coupling feature in which it is positioned again above inner pockets between inner portions and not on the top faces of outer portions anymore. |

Reference may be made to a cylindrical coordinate system, i.e. each position may be defined by three coordinates: axial value (height, distance to zero plane), radial distance to axis and angle between current radial position and a plane that is defined as having angle zero. In this document the words "in an axial position" may mean having an axial coordinate.

The distal end D may be an end that is closer to a needle compared to a proximal end P.

Certain embodiments in this document are illustrated with respect to an injection device where an injection button and grip (dose setting member or dose setter) are combined. Reference is made to WO 2014/033195 A1 or to WO 2014/033197 A1 in this regard which are included by reference for all legal purposes. The injection button may provide at least one user interface member for initiating and/or performing a dose delivery operation of the drug delivery device. The (dial) grip or knob may provide a user interface member for initiating and/or performing a dose setting operation using a dose setting surface, e.g. the circumferential surface of the (dial) grip or knob. A delivery surface may be used to initiate dose delivery. The delivery surface may be the proximal P surface of the (dial) grip or knob.

The device may be of the dial extension type, i.e. its length increases during dose setting or dose dialing. Other injection devices with the same kinematical characteristic of the dial extension and button during dose setting and dose expelling operational mode are known as, for example, Kwikpen® and Savvio® device marketed by Eli Lilly (see e.g. WO2005/018721) as well as FlexPen® (see e.g. US6,004,297), FlexTouch® (see e.g. US9,108,002) and Novopen® 4 (see e.g. 5,693,027) device marketed by Novo Nordisk or devices of other manufacturers. An application of the general principles to these devices therefore appears straightforward and further explanations will be omitted. Alternatively, the proposed concepts may be used in devices that are not of the dial extension type but include for instance torsion spring that are biased by rotation of a dial knob. Moreover, fully electromechanical drug delivery devices may be used, e.g. comprising an electrical motor.

However, the general principles of the present disclosure are not limited to that kinematical characteristic. Certain other embodiments may be conceived, e.g. for application to other injection devices of Sanofi or devices of other manufacturers where there are e.g. separate injection button and grip components / dose setting members. Thus, there may be two separate user interface members, one for the dose setting operation and one for the dose delivery operation.

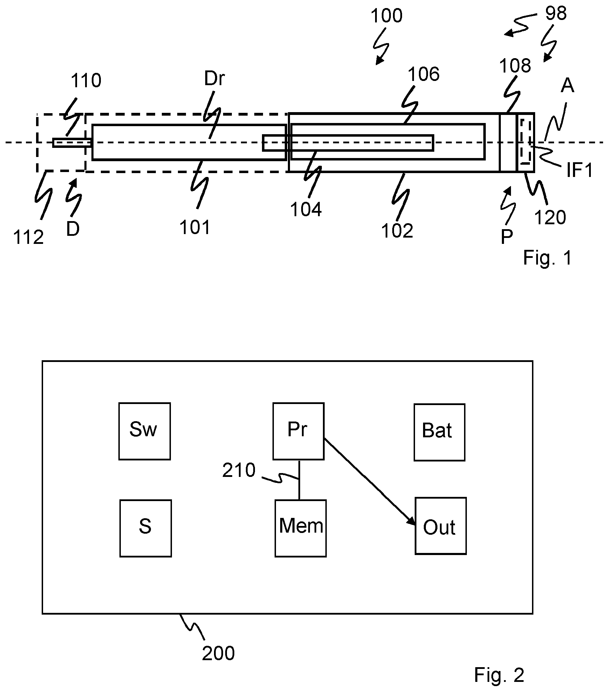

Figure 1 illustrates an electro-mechanical system 98 according to a first embodiment. Electro-mechanical system 98 may comprise a drug delivery device 100 that may comprise a container retaining member 101 retaining a drug container (e.g. a syringe or a cartridge) and a main housing part 102. The container may comprise a drug Dr. Main housing part 102 may house or surround the container retaining member 101 and/or the container. completely or partially and may comprise further parts of the drug delivery device 100. Alternatively, the main housing part 102 may be connected to the container retaining member 101 but may not surround it and even may not surround a part of the container retaining member 101, see dashed line in Figure 1.

A longitudinal axis A of drug delivery device 100 is illustrated in figure 1 by a dashed line.

Within the main housing part 102 the following components may be arranged:

- A piston rod 104 that is adapted to move a piston that may be arranged within container retaining member 101,

- A driving mechanism 106 for the piston rod 104. The driving mechanism 106 may comprise an energy storing element, for instance a spring that is loaded manually before each use. Alternatively, the energy storing element may be loaded for instance during assembling of drug delivery device 100. Alternatively, a manually driven driving mechanism may be used, e.g. without an energy storing element that is used to drive piston rod 104.

- For instance at a proximal end P, an actuating element 108 that is used for the initiation of a movement of piston rod 104 into the container retaining member 101, whereby the driving mechanism 106 is used. Alternatively, an autoinjector device may be used that is actuated by an axial movement of a movable needle shroud (not shown). Actuating element 108 may be used to dial the size of a dose of drug Dr in some embodiments.

- A cap 112 that may be attached to main housing part 102 or to another part of drug delivery device 100. Cap 112 may be an outer cap that may include a smaller inner cap which protects a needle 110 directly.

If drug delivery device 100 is not an autoinjector, a dial sleeve may be screwed out of main housing 102 and may be pressed by a user in order to move plunger 104 distally and to inject drug Dr. Drug delivery device 100 may be a single use or a multiple use device.

Drug Dr may be dispensed from the container through needle 110 or through a nozzle that is connectable and/or connected to the distal end D of drug delivery device 100. Needle 110 may be changed before each use or may be used several times.

System 98 may comprise an electronic unit 120 that is mechanically connected to a proximal end region P of drug delivery device 100, for instance to a proximal end region P of actuating element 108. Electronic unit 120 may have a longitudinal axis A that is the same axis as the longitudinal axis A of drug delivery device 100.

Electronic unit 120 may be used not only for drug delivery device 100 but also for other drug delivery devices that are similar or identical to drug delivery device 100. Thus, electronic unit 120 may be a module used multiple times with different drug delivery devices 100, etc. in different systems 98, etc. Furthermore, the diameter of drug delivery device 100 is not increased by electronic unit 120 promoting excellent handling of modular system 98, and especially of drug delivery device 100.

Alternatively, electronic unit 120 may be an integrated part of the drug delivery device 100 which may be identical to electro-mechanical system 98 in this case. That is to say, that electronic unit 120 may be used together with only one drug delivery device 100 that may be re-usable or may be only a single use device.

Electronic unit 120 may comprise an interface unit IF1 that is appropriate to convert a rotation of a rotating member of the drug delivery device 100 into an axial and/or a radial displacement of a coupling portion. Interface unit IF1 may be similar as interface unit IF2 described in more detail below. Alternatively, interface unit IF1 may have a different construction, considering e.g. the modifications already mentioned in the first part of the description or mentioned in the following.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir®); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba®); B29-N-(N-lithocholyl-gamma-glutamyl)-des(B30) human insulin; B29-N-($\omega$-carboxyheptadecanoyl)-des(B30) human insulin and B29-N-($\omega$-carboxyheptadecanoyl) human insulin.

Examples of GLP-1, GLP-1 analogues and GLP-1 receptor agonists are, for example, Lixisenatide (Lyxumia®), Exenatide (Exendin-4, Byetta®, Bydureon®, a 39 amino acid peptide which is produced by the salivary glands of the Gila monster), Liraglutide (Victoza®), Semaglutide, Taspoglutide, Albiglutide (Syncria®), Dulaglutide (Trulicity®), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro®), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc®), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

Figure 2 illustrates schematically an electronic unit 200, for instance electronic unit 120. Electronic unit 200 may comprise:

- At least one processor Pr or another control unit,
- A memory Mem, for instance volatile and/or nonvolatile storing memory,
- A battery Bat or a rechargeable accumulator or any other electrical power source,
- An output device Out, for instance a transmitting (sending) unit, for instance for communication with a smartphone or other computer device. Alternatively or additionally, output device Out may comprise a display unit, e.g. an LCD (Liquid Cristal Display) unit or another appropriate unit.
- An optional input device In, for instance for communication with a smartphone or other computer device. Input device In may receive confirmation commands and/or data, especially user data,
- A switch Sw that may be used for waking up electronic unit 200 from a first energy state with reduced energy consumption to a second energy state having higher energy consumption compared to the first energy state, and
- At least one sensor S or at least two sensors, preferably optical sensor(s) that may be arranged near a rotating encoder part and that may be configured to detect rotation of the rotating encoder parts. Rotation of the encoder part may be proportional to drug Dr dialed or delivered by drug delivery device 100.

Further parts may be comprised in electronic unit 200 that are not shown, for instance a radiation source, especially a light source or another radiation source, e.g. IR (infrared). A data transmission bus 210 may connect processor Pr an memory Mem.

Processor Pr may be a microcontroller or microprocessor that performs instructions of a program which is stored in memory M. Alternatively, an FPGA (Field Programmable Gate Array), ASIC (Application Specific Integrated Circuit), PLA (Programmable Logic Array), PLD (Programmable Logic Device) or another appropriate circuitry may be used to implement a finite state machine that does not perform instructions of a program.

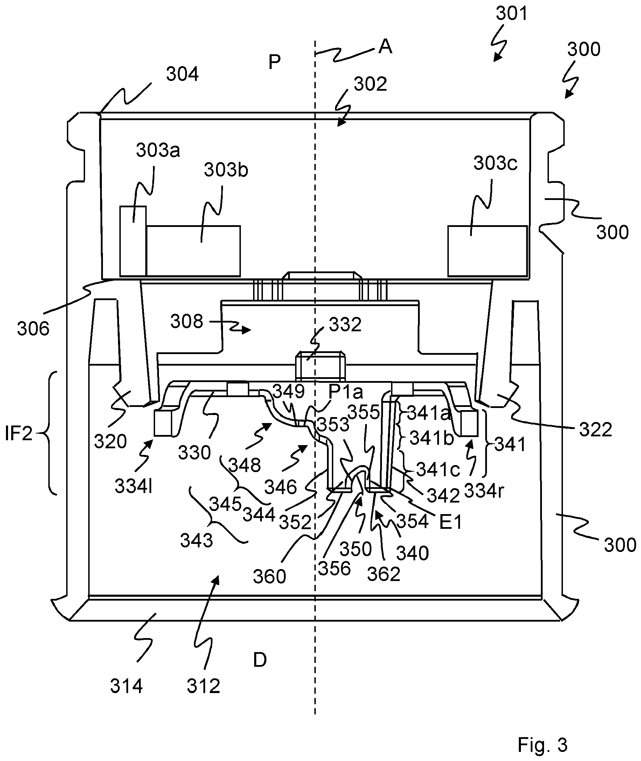

Figure 3 illustrates a cross section of a button inner 300 (e.g. chassis), showing an interface unit IF2 comprising e.g. a coupling feature 341 (comb-like feature) on a flexible arm or ring 330.

Wake switch embodiments are described e.g. for a clip-on electronic encoding unit/module 120, 200. The present disclosure relates to features of an electronic unit/module 120, 200 that can be embodied as a re-usable clip-on module with a suitably configured pen injector for the purpose of recording doses that are delivered from the pen or other drug delivery device 100. This functionality may be of value to a wide variety of device users as a memory aid or to support detailed logging of dose history data. It is envisaged that electronic unit/module 120, 200 could be configured to be connectable to a mobile device, e.g. a smart phone, or similar, to enable the dose history data to be downloaded from unit/module 120, 200 on a periodic basis.

In an embodiment, the wake switch Sw may be driven by rotation of the dose delivery mechanism within the injection device 100. In the device as described in WO 2014/033195 A1 or in WO 2014/033197 A1 this rotation may be observed in a number sleeve 400, which may be configured to rotate relative to a dose button during dispense, but e.g. not during dose-setting (dialing). The dose button may comprise button inner 300. Both documents WO 2014/033197 A1 are enclosed by reference herewith for all legal purposes.

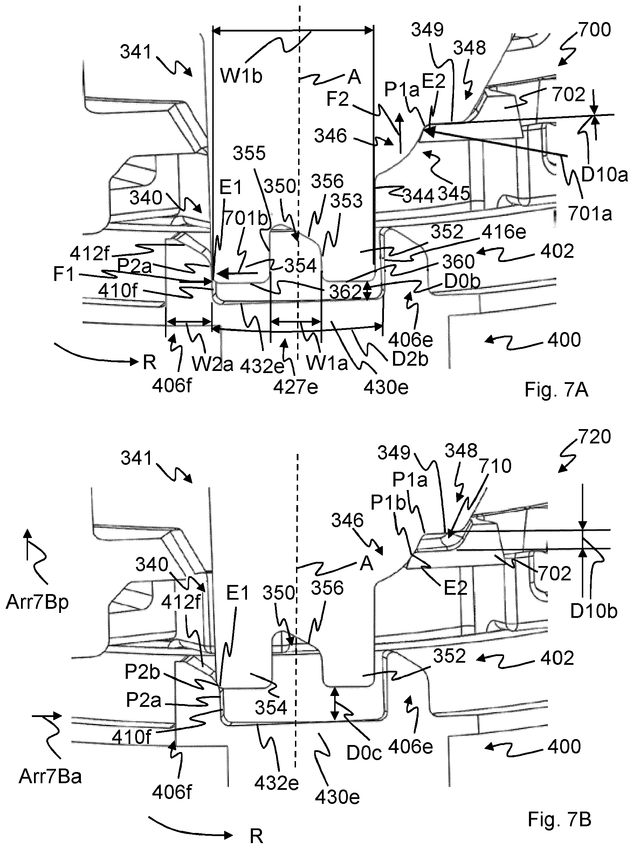

Button inner 300 may form a chassis, e.g. a light pipe chassis or another chassis. Button inner 300 may comprise a flexible switcher arm feature 330, 334l, 334r. The switcher arm feature 330, 334l, 334r may comprise a two-pronged coupling portion 340, e.g. 'comb' shape, which may be configured to such that it does not close switch Sw, e.g. a microswitch, due to axial contact with number sleeve 400 during axial travel of the dose button, e.g. button inner 300, see figure 3. Figure 3 illustrates coupling portion 340 with two prong shape, e.g. front coupling portion 352 and back coupling portion 354, and a rotational bearing surface 345 adapted to abut against a ramped feature 702, see figures 7A to 7D, of a button inner 300 forming e.g. a chassis. Figure 3 is a cross section of button inner 300, e.g. of a light pipe chassis or other chassis showing coupling portion 340 on flexible arm, e.g. holding ring 330. The optional light pipes are not illustrated in figure 3.

Button inner 300 may be part of an actuating unit 301 as mentioned in the first part of the description. Actuating unit 301 may comprise interface unit IF2. Interface unit IF2 may be similar to interface unit IF1 or may have different inner construction.

Button inner 300 may comprise:

- An upper chamber 302 (compartment), e.g. for electronic components 303a, 303b and 303c of an electronic circuit. The electronic circuit may be mounted and connected by a circuit carrier, e.g. a printed circuit board (PCB) that is not shown. The electronic components 303a, 303b and 303c may be capacitor(s), inductor(s) and/or communication module and or other parts of electronic unit 120, 200, and/or
- A proximal opening 304, e.g. for a drug delivery button or for a proximal P cap, and/or
- An inner circumferential ledge 306, e.g. for supporting a PCB or other electric circuit carrier, and/or
- A cavity 308 for switch Sw, e.g. micro switch (not shown), and/or
- A lower chamber 312 (compartment), e.g. for interface unit IF2, and/or
- A distal opening 314, e.g. for inserting a rotating member (ring member 402) of drug delivery device 100, and/or

- Resilient hooks 320, 322 (extending e.g. from circumferential ledge 306), e.g. for clipping actuating unit 301 to drug delivery device 100, and/or
- A holding ring 330, e.g. circumferential direction of holding ring 330 may correspond to circumferential direction of button inner 300, and/or
- An actuation portion 332, e.g. a protruding region that protrudes from holding ring 330, e.g. from proximal parts of coupling feature 341, into cavity 308, and/or
- A fastening region of ring is not illustrated and may be arranged opposite actuation portion 332 on holding ring 330, and/or
- A left portion 334l of holding ring 330. Left portion 334l may have the function of a retaining element. Alternatively, a straight retaining element may be used that extends e.g. along the diameter of holding ring 330. However, curved arms may allow positioning of other parts within center of holding ring 330, and/or
- An optional right portion 334r of holding ring 330.

Button inner 300 may comprise a coupling portion 340. Coupling portion 340 may be comprised in a coupling feature 341. Coupling feature 341 may be hold by holding ring 330.

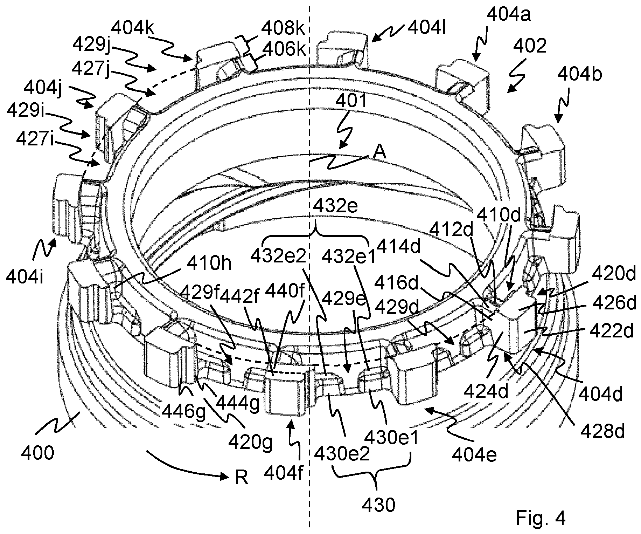

Coupling feature 341 may comprise a proximal portion 341a and/or a middle portion 341b and/or a distal portion 341c. Distal portion 341c may be essentially identical with coupling portion 340. Coupling feature 341 may comprise a back face 342 (vertical), e.g. backwards facing or backwards directed with regard to the direction R of rotation of a ring member 402, see figure 4. Coupling feature 341 may comprise a front face 343 relative to the direction R of rotation of a ring member 402.

Front face 343 of coupling feature 340 may comprise:

- A vertical portion 344, e.g. frontwards facing or frontwards directed, and
- A curved portion 345 of front face 343.

Curved portion 345 of front face 343 may comprise:

- A distal curved portion 346, and
- A proximal curved portion 348.

A point P1a may be arranged between curved portions 346 and 348. Point P1a may be arranged on a concave edge of coupling feature 341. Furthermore, there may be a distally facing portion 349 on second curved portion 348.

Coupling portion 340 may comprise:

- A recess 350 also named as recessed portion, and/or
- A front coupling portion 352 (prong, claw), and/or
- A back face 353 of front coupling portion 352 (front face of recess 350, backwards facing/directed), and/or
- A back coupling portion 354 (prong, claw), and/or
- A front face 355 of back coupling portion 354 (back face of recess 350, frontwards directed).

A distal face 360 may form the distal end of front coupling portion 352. A distal face 362 may form the distal end of back coupling portion 354. There may be an edge E1 formed between distal face 362 of back coupling portion 354 and vertical back face 342 of coupling portion 340. Edge E1 is used to push the coupling feature proximally as is explained in more detail below, see figures e.g. 7A to 7E.

Figure 4 illustrates ramped features 412d on teeth 404a to 404l of a number sleeve 400, more specific of a ring member 402 on a proximal end of number sleeve 400. Number Sleeve 400, e.g. of the pen device as described in WO 2014/033197 A1 or of another drug delivery device 100, may be configured to have ramped features, e.g. 412d, on each of the teeth, e.g. 404d, e.g. teeth of a clutch. There may be for instance 12 teeth 404a to 404e, see figure 4. Furthermore, there may be flat surfaces or faces 426d on each of the teeth 404a to 404e, see for instance tooth 404d, e.g. in order to prevent that the coupling portion 340 (comb features) slides back into the inner pockets 427i, 427j between the teeth 404i, 404k. Exemplary, WO 2014/033195 A1, figure 4, clutch 90 has teeth that are outwards directed. Teeth at the inner of proximal end of dial sleeve 62 are inwards directed. Contrary, in this application, teeth 404a to 404l on number/dial sleeve 400 are directed radially outwards. Thus the "clutch" of this embodiment, i.e. ring member 402 is different from the clutches.

Exemplary, in the WO document WO 2014/033195 A1, the teeth of the number sleeve/dial sleeve are directed radially inward and the teeth of the mating button component (or 'clutch') are directed outward. In the concept described in this document, the direction of teeth is swapped between the two components, so teeth are directed outward on the number sleeve and directed inward on the button. However, the clutch mechanism works in the same way as in the device described in the WO documents 2014/033195 A1, WO 2014/033197 A1 - the clutch system still rotationally couples the button and number sleeve (via radial teeth) when the button is moved into a certain relative axial position, and rotationally decouples them in other relative axial positions. However, other clutch coupling mechanism may be used as well. Alternatively, no clutch mechanism may be involved.

So, the clutch mechanism is based on the same design concept as in the WO document, but is a slightly different embodiment.

During dose delivery, number sleeve 400 and/or dial sleeve 400 may rotate, and the ramp features, e.g. 412d, of number sleeve 400 and/or dial sleeve 400 may engage with one of the two prongs (front coupling portion 352 and back coupling portion 354) on the coupling portion 340 of button inner 300, e.g. of a light pipe chassis or a chassis for other sensor units. This in turn may cause the flexible coupling portion 340 to bear rotationally against a ramped feature 702 on a button hold (button inner 300) within the chassis or on the chassis itself and to deflect upwards relative to the rest of the dose button and electronic module/unit 120, 200, thereby driving e.g. switch Sw axially, e.g. a micro-switch. Switch Sw may wake-up the micro-controller or another processor Pr and may initialize e.g. IR-LEDs (Infrared Light emitting Diode) of the dose capture system. Switch Sw or sensor may preferably have a very light operating force, to ensure that the load to operate it does not apply a large drag torque to the number/dial sleeve 400 during dispense, which could manifest in a high dispense force for the user.

As the coupling portion 340 is deflected upwards by the rotation of the number/dial sleeve 400, it may also deflect radially outwards. This may allow coupling portion 340 to bridge between adjacent teeth 404a to 404l on the number/dial sleeve 400, at a radius which may be larger than the radius at which the ramp features, e.g. 412d, are formed. This may act to allow the coupling portion 340 to ride over flat sections, e.g. 426d of the number/dial sleeve 400 during dose dispense, and thereby apply a low level of drag torque.

At the end of dispense, the user may release the dose button, e.g. button inner 300, which may spring axially outwards from the pen, e.g. in proximal direction. This may allow the coupling portion 340 on the flexible arm(s) 334l, 334r of the button inner 300 and/or chassis to once again deflect radially inwards and downwards, and switch Sw or another sensor may open or fulfill another switching operation.

Dose synchronization and/or Bluetooth (e.g. Bluetooth LE (low energy)) or other small distance radio pairing may be achieved with an additional switch elsewhere on the electronic module. E.g. an axial switch on the top surface of the module. If the additional switch is held closed for a specified period of time, e.g. 3 to 5 seconds, dose synchronization may be induced. Dose synchronization may include updating the list of injected doses and/or time and date of injection. If the additional switch is held closed longer, e.g. 5 seconds or more than five seconds Bluetooth pairing or another pairing protocol may be started. Pairing may refer to the first coupling of two devices in order to establish a communication connection between these two devices.

Number sleeve 400 may comprise circular central opening 401 and a coupling ring member 402 on its proximal end portion. Coupling ring member 402 may comprise teeth 404a to 400l, e.g. 12 teeth, arranged around the circular opening 401 and supported by a central ring of ring member 402.

Teeth 404a to 400l, e.g. tooth 404d, may comprise:

- An inner portion 406a to 406l, e.g. 406k, that may form a sub-tooth of tooth 404a to 404l and that may have a specific function, e.g. the functions of a crown tooth that is appropriate for axial engagement, and/or,
- An outer portion 408a to 4081, e.g. 408k, that may form at least one further sub-tooth or at least two further sub-teeth, having functions different to the function of the first sub-tooth, e.g. a holding function as explained below and/or a function of a spur tooth.

There may be the following faces on an inner portion of tooth, e.g. of tooth 404d:

- A vertical front face, e.g. 410d, and/or
- A front ramped face, e.g. 412d, and/or
- An optional back ramped face, e.g. 414d, and/or
- A vertical back face, e.g. 416d, and/or
- A bottom area, e.g. 418d.

There may be the following faces on outer portions, e.g. on outer portion of tooth 404d:

- A front face 420d, e.g. arranged parallel to the longitudinal axis A of drug delivery device 100, e.g. rotation axis A of number sleeve 400, and/or
- A side face 422d, e.g. parallel to the longitudinal axis A, and/or
- A back face 424d, e.g. parallel to the longitudinal axis A, and/or
- A top face 426d, e.g. arranged cross to the longitudinal axis A and/or

- A bottom face 428d, e.g. cross to the longitudinal axis A.

Furthermore, there may be inner pockets, e.g. 427d, 427e, 427f between two adjacent inner portions, see 406k. Outer pockets 429d, 429e, 429f may be arranged between two outer portions, see 408k.

Vertical back face 416d of inner portion of a respective tooth, e.g. 404d, and back face 424d of outer portion may be arranged within the same plane, e.g. both back faces, e.g. 416d and 424d, may be coplanar to each other. However, other arrangements may also be used.

At least one intermediate portion(s) 430e may be arranged at the bottom of inner pockets between two adjacent inner portions, e.g. 406k, e.g. a right intermediate portion 430e1 and a left intermediate portion 430e2. Proximal face(s), e.g. 432, of the at least one intermediate portion(s), e.g. 430e, may face proximally P. Thus, there may be a proximal face 432e1 of right intermediate portion 430e1 and a proximal face 432e2 of left intermediate portion 430e2.

The inner sub-portions, e.g. 440f, of outer portions, e.g. 408f, may have the function of a holding structure preventing re-engagement of coupling portion 340 with inner teeth 406a to 406l under specific conditions that are described in more detail below. Outer sub-portions, e.g. 442f, of the outer portions, e.g. 408f, may have a different function compared to the function of inner sub-portions, e.g. 440f. Thus, outer sub-portions, e.g. 442f, may have the function of clutch teeth of a clutch for forwarding a rotational movement between different parts or for disabling forwarding of a rotational movement between these different parts of the clutch.

Proximal (top) faces of inner sub-portions, e.g. 440f, and outer sub-portions, e.g. 442f, may be arranged within the same plane, i.e. be coplanar to each other, see for instance face 426d. However, other arrangements of these two proximal faces may also be used.

There may be a leading edge, e.g. 444g, on each of the front faces, e.g. 420g, of an outer portion, e.g. 408k. The leading edge, e.g. 444g, may be convex. Furthermore, there may be a concave edge 446g of the front face, e.g. 420g, at a border between an inner sub-portion, e.g. 440g, and an outer sub-portion, e.g. 442g on the front face, e.g. 420d, of an outer portion, e.g. 408d, 408k. The concave edge, e.g. 446g, may form the border between an inner part of front face, e.g. 420g, and an outer part of front face, e.g. 420g. Inner part of the front surface or face, e.g. of front face 420g may be curved. An axis of curvature may be arranged parallel to longitudinal axis A. An outer part of the front surface, e.g. 420g, may be flat. Figure 4 illustrates a direction of rotation R relative to which the terms "front" and "back" are used.

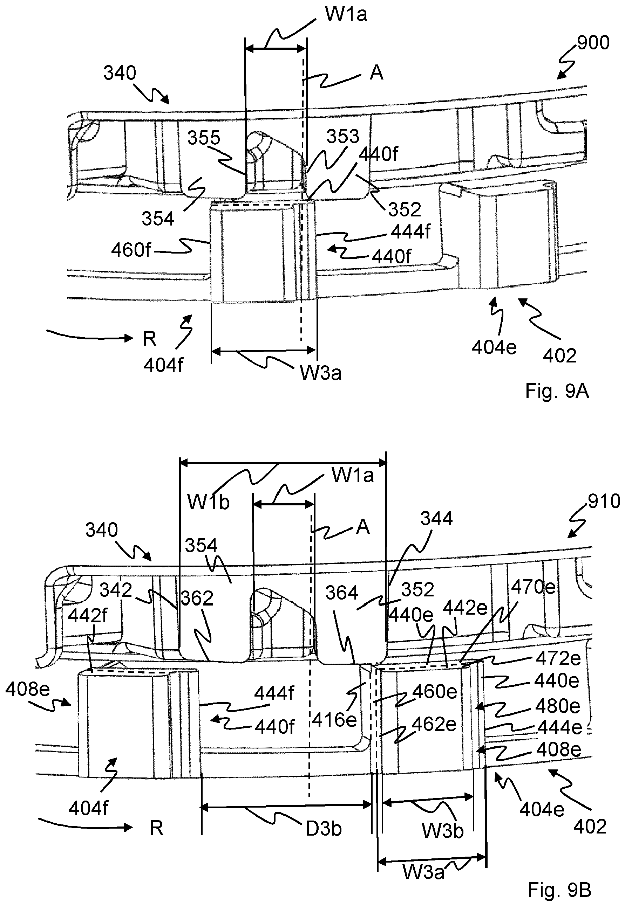

As is illustrated in more detail in figure 9B a back face, e.g. 460e, of inner sub-portion, e.g. 440e, and a back face, e.g. 462e, of an outer sub-portion, e.g. 442e, on the same tooth, e.g. 404e, may be arranged within the same plane. However, other arrangements of these faces are possible as well.

A top face, e.g. 470e, see figure 9B, of an inner sub-portion, e.g. 440, may be arranged within the same plane as a top face, e.g. 472e, of an outer sub-portion, e.g. 442e, on the same tooth, e.g. 404e. However, other arrangements of these faces are possible as well. A front face, e.g. 480e, of outer sub portion 408e is also illustrated in figure 9B that is described in more detail below.

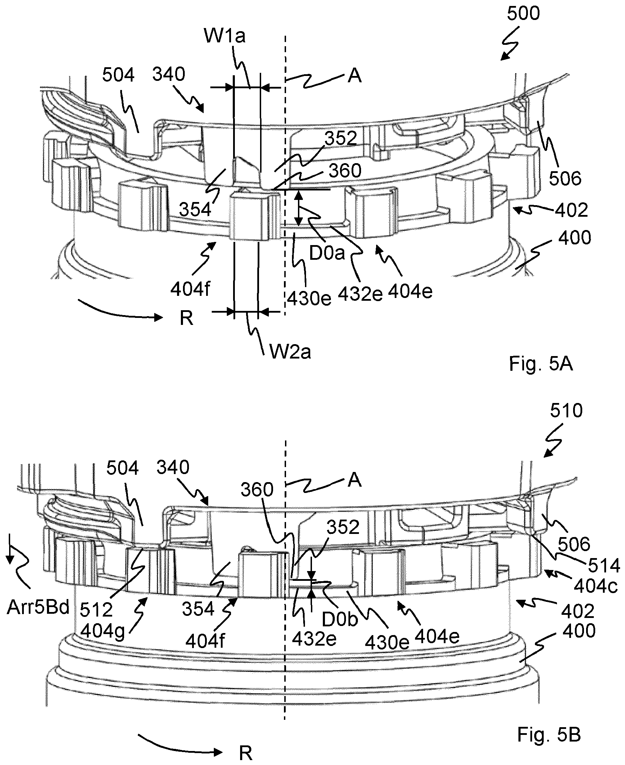

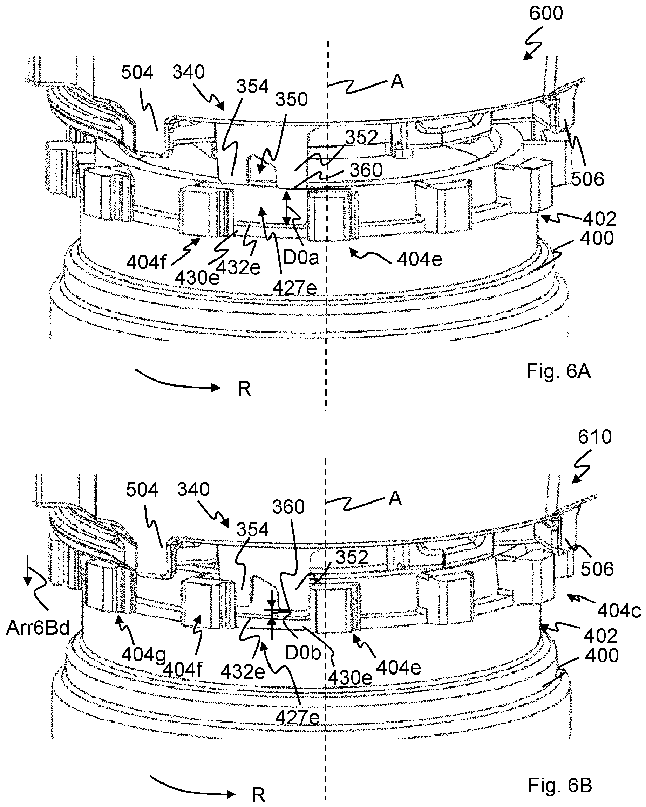

Figures 5A and 5B illustrate coupling feature 340 as the button inner 300 travels axially, e.g. distally, straddling a single tooth 404f, e.g. the seventh tooth of ring member 402. As there may be less teeth than separate positions for ending the dosing (dose delivery) operation, e.g. only 12 teeth, but twice as much, e.g. 24, separate positions, coupling feature 340 may need to have at least two protrusions 352, 354 so that it can drop down into engagement with teeth 404a to 404l of the number/dial sleeve 400 in both conditions, i.e. either straddling a single tooth, e.g. 404f, see figures 5A and 5B, or between teeth, see figures 6A and 6B.

Figure 5A illustrates a configuration 500 comprising a first axial position and a first angular position of button inner 300 (button inner) and of coupling portion 340 relative to ring member 402 and number sleeve 400. There may be a first axial distance D0a between a distal face 360 of front coupling portion 352 and a proximal face 432 of intermediate portion(s) 430e. Distance D0a may be equal to the axial length of inner portion(s), e.g. 406f.

The circumferential or angular width W1a of recess 350 may be slightly larger than the circumferential or angular width W2a of each inner portion, e.g. 406f. Thus, recess 350 and therefore also coupling portion 340 may engage with inner teeth, e.g. 406f, see figure 5A. There may be optional portions 504, 506. Front ramped faces, e.g. 412f, may fulfill a distal stop function for recess 350, e.g. for coupling portion 340. Furthermore, proximal faces, e.g. 432e, 432f, of intermediate portion(s), e.g. 430e, 430f, may also be used as a distal stop face for the distal movement of button inner 300, e.g. of coupling portion 340.

Figure 5B illustrates a second configuration 510 comprising a second axial position and the first angular position of button inner 300 (button inner) and of coupling portion 340 relative to ring member 402 and number sleeve 400. An axial distance D0b between distal face 360 of front coupling portion 352 and proximal face 432 of intermediate portion(s) 430e is less than axial distance D0a because button inner 300 has been moved distally by a user, see arrow Arr5Bd. Distance D0b may be greater than zero millimeter or may be zero millimeter. However, due to the engagement of recess 350 and inner tooth of tooth 404f the axial movement of coupling member 340 is possible and no blocking or deflection of coupling portion 340 occurs, e.g. actuating portion 332 does not act on switch Sw.

Figures 6A and 6B illustrate coupling feature 340 as the button 300 travels axially (distally), between two teeth 404e and 404f of ring member 402 of number sleeve 400 thereby straddling no tooth. A configuration 600 illustrates the first axial and a third angular position of button inner 300 (button inner) and of coupling portion 340 relative to number sleeve 400.

Coupling portion 340 is arranged above an inner pocket 427e between teeth 404e and 404f. There may be a first axial distance D0a between a distal face 360 of front coupling portion 352 and a proximal face 432 of intermediate portion(s) 430e. Distance D0a may be equal to the axial length of inner portion(s), e.g. 406f.

A further configuration 610 comprises the second axial and the third angular position of button inner 300 (button inner) and of coupling portion 340 relative to number sleeve 400. An arrow Arr6Bd illustrates the distal movement of button inner 300 relative to number sleeve 400.

Coupling portion 340 is now engaged in inner pocket 427e. An axial distance D0b between distal face 360 of front coupling portion 352 and proximal face 432 of intermediate portion(s) 430e is less than axial distance D0a. Distance D0b may be greater than zero millimeter or may be zero millimeter. Proximal face, e.g. 432e, of intermediate portion(s), e.g. 430e, may be used as a distal stop face for the distal movement of button inner 300 in the variant that is illustrated in figures 6A and 6B.

However, due to the engagement of coupling portion 340 between teeth 404e and 404f the axial movement of coupling member 340 is possible and no blocking or deflection of coupling portion 340 occurs, e.g. actuating portion 332 does not act on switch Sw in the variant that is illustrated in figures 6A and 6B.

Figure 7A illustrates a first configuration 700 of ring member 402, coupling feature 340 and ramped feature 702. Configuration 700 illustrates the axial and angular positions of these parts 402, 340 and 702 relative to each other. Figure 7A illustrates a cross section through coupling feature 340 (comb) showing engagement of coupling feature 340 with ring member 402 of number sleeve 400, e.g. within inner pocket 427e that is located between inner portions 406e and 406f that form teeth 406e and 406f.

Taking a section through the comb-like feature 340 in Figure 7A we can see that coupling feature 341 is contacting both a number sleeve tooth 406f on one side (left) and an optional ramped feature 702 on the button inner 300 (chassis) on the other (right) side, e.g. on front face 343 of coupling feature 341, more specifically on curved portion 345 of front face 343.

Figure 7A illustrates the following geometrical dimensions:

- Circumferential or angular width W1a of recess 350,
- Circumferential or angular width W1b of distal portion 340c, especially at its distal end,
- Circumferential distance D2b between opposite faces of two adjacent inner portions 406e, 406f, and
- Circumferential or angular width W2a of inner portion(s), e.g.406f.

The following equation may be valid:

$$W1a > W2a, (1),$$

wherein W1a is the angular width of recessed portion 350 of the coupling portion 340, and
wherein W2a is the angular width of an inner tooth, e.g. 406e, 406f, of the rotating member 402.

Thus, recess 350 can engage with inner tooth 406f.
Further, the following equation may be valid.

$$D2b > W1b, (2),$$

wherein D2b is the angular distance between a back face, e.g. 416e, of a first one of the inner tooth, e.g. 404e, and the front face, e.g. 410f, an adjacent inner tooth, e.g. 404f, and
wherein W1b is the angular width of the coupling portion 340. Thus, coupling portion 340 can engage with inner pockets, e.g. 427e between inner portions, e.g. 406e and 406f, that may form inner teeth, e.g. 406e and 406f.

The same operations that are explained in the following for the movement of coupling portion 340 (coupling feature 341) and ramped feature 702 remain valid for the case in which recess 350 and an inner portion 406f (inner tooth) are engaged and a rotation force of ring member 402 acts on back face 353 of front coupling portion 352.

A circumferential or angular force F1 may be generated by rotation of ring member 402 by inner tooth 406 onto the back edge E1 of coupling member 340. As is explained in the following in more detail, angular force F1 is converted to a proximal movement of coupling feature 341, see e.g. arrow Arr7Bp in figure 7B.

Further referring to figure 7A, an arrow 701a indicates the contact portion between curved portion 345 of coupling feature 341 and ramped feature 702. An arrow 701b illustrates the contact portion between coupling portion 340 and inner tooth 406f.

Guiding feature or ramped feature 702 on button inner 300 may comprising at least one ramp, e.g. a linear ramp or a curved ramp as described in more detail below. Ramped feature 702 may be arranged fixed relative to button inner 300 (chassis), e.g. ramped feature 702 may not rotate but is rotationally fixed on button inner 300 that does also not rotate but may move axially.

Other possibilities for using curved features on coupling feature 341 and/or on ramped feature 702 that may be named as guiding feature are e.g.:

- Curved face (surface) only on coupling feature 341 but not on guiding feature 702, e.g. guiding feature may be a round pin. The round pin may interact with curved face 345 on coupling feature 741.
- Curved face (surface) only on guiding feature 702 but not on coupling feature 341, e.g. only a round pin on coupling feature 741 may be used. The round pin on coupling feature 741 may interact with curved face 710 on guiding feature 702.

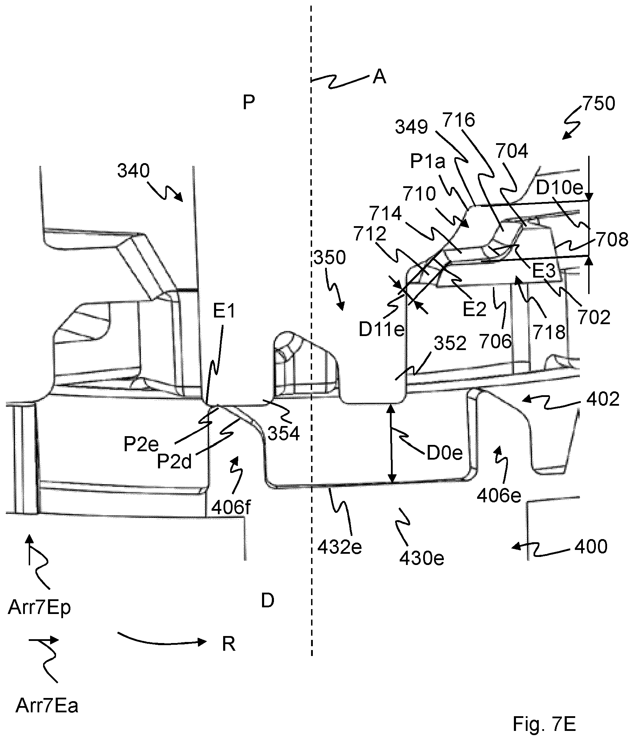

There may be the following faces on ramped feature 702, see also figure 7E:

- A proximal (top) face 704, and/or
- A distal (bottom) face 706, and/or
- A front face 708 (e.g. relative to rotation of ring member 402, and/or
- A curved back face 710, and/or
- An outer face 718, and/or
- An inner side area between ramped feature 702 and button inner 300.

There may be the following portions of curved back face 710, see also figure 7E:

- A distal portion 712 facing backwards, and/or
- A middle portion 714 facing proximally, and/or
- A proximal portion 716 facing backwards.

An edge E2 may be formed by the border between distal portion 712 and middle portion 714. Edge E2 may be a convex edge. An edge E3 may be formed by the border between middle portion 714 and proximal portion 716. Edge E3 may be a concave edge.

In the first configuration 700 curved face 345 and curved face 710 may be engage complementary, e.g.:

- Distal portion of distal curved portion 346 may be opposite and/or in (direct) mechanical contact to distal portion 712,
- Convex edge E2 may contact concave edge between curved portion 346 and 348, see point P1a,
- Middle portion 714 may directly contact distal facing portion 349,
- Proximal portion 716 may be arranged opposite to a more proximal portion of curved portion 348 compared with distal facing portion 349. There may be direct mechanical contact between these two portions or there may be a small distance.

A point P2a is located on vertical front face 410f of inner portion/tooth 406f. Point P2a may be in direct mechanical contact with edge E1 on coupling portion 354. Thus, the force F1 impacted on point P2a may be forwarded via coupling feature 341 to point P1a and then to ramped feature 702. Ramped feature 702 may generate a proximal directed force F2 due to its complementary contact with curved portion 345. Resiliency of coupling feature 341 and/or resilient holding of coupling feature 341 on arms (ring portion) 334l and 334r may promote the conversion of force F1 to force F2.

A distance D10a between middle portion 714 of curved face 710 on ramp feature 702 and distally facing segment 349 of curved portion 348 corresponds essentially to the axial distance between edge E2 and point P1a. In configuration C1, 700 distance D10a may be zero millimeter.

A distance D0b between distal face 360 of front coupling portion 352 and proximal face 432e of intermediate portion 430e may have the value mentioned above, e.g. of more than zero millimeter as illustrated in figure 7A or of zero millimeter.

Figure 7B illustrates a second configuration 720 of ring member 402, coupling feature 340 and ramped feature 702. Figure 7B illustrates a cross section through coupling feature 340 (comb) showing the coupling feature 340 to ride up the button inner 330, e.g. to ride up on ramped feature 702 of button inner 330.

As number sleeve 400 rotates, e.g. moving from left to right in figures 7A to 7E, it may deflect coupling portion 340 to the right, driving coupling portion 340 up the angled/curved face 710 on the ramped feature 702 of button inner 300 (chassis) to open up the gap D10b shown in figure 7B. The angle of this face 710 may start steep and may gradually get less steep as coupling portion 340 drives up this face. This may be because the force required to deflect coupling feature 340 elastically may increase as coupling portion 340 moves up axially. This may be in part due to the actuation force needed for actuating switch Sw. Therefore, as this angle gets shallower the overall torque required from the number/dial sleeve 400 to drive this action may be reduced and may remain approximately constant during the motion. The torque to drive number/dial sleeve 400 may be generated by the user applying axial force to number/dial sleeve 400 and this force may be reacted by a helical thread interface of number/dial sleeve 400. Therefore, reducing this torque may reduce the additional force required by the user to initiate dispense.

A distance D10b between middle portion 714 of curved face 710 on ramp feature 702 and distally facing segment 349 of curved portion 348 may now be greater than distance D10a. A distance D0c between distal face 360 and proximal face 432e of intermediate portion 430e may now be greater than distance D0b.

Edge E2 has reached and/or makes direct mechanical contact with a point P1b which is located distal on distal curved portion 346 compared to point P1a. Edge E1 has reached and/or makes direct mechanical contact with a point P2b that is more proximal that point P2a on front face 410f of inner tooth 406f.

An arrow Arr7Bp illustrates the proximal displacement of coupling feature 341 comprising coupling portion 340. An arrow Arr7Ba illustrates the angular movement of ring member 402.

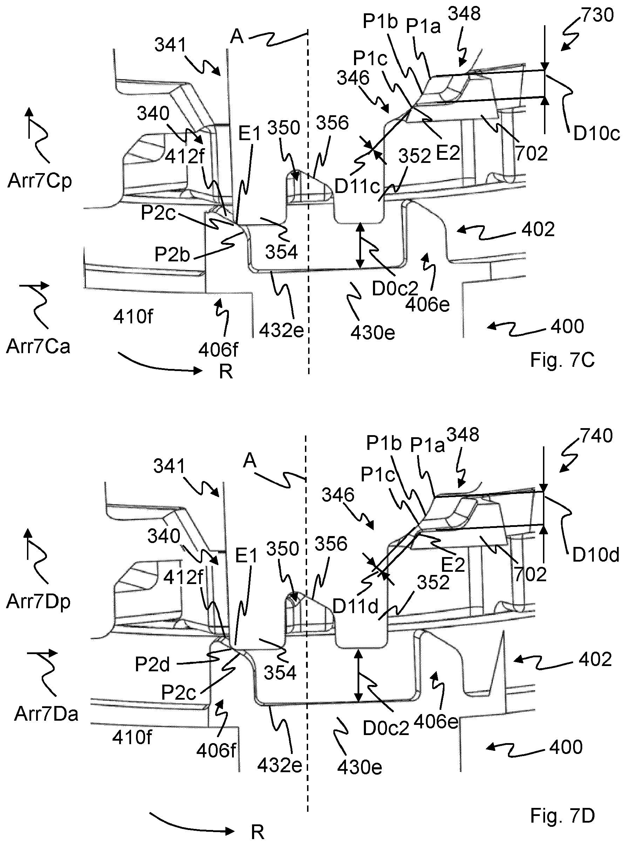

Figure 7C illustrates a third relative configuration 730 of ring member 402, coupling feature 340 and ramped feature 702. Figure 7C illustrates a section through coupling feature 340 (comb), showing coupling feature 340 beginning to ride up number sleeve 400 tooth angled face, e.g. angled face or ramped face 412f of tooth 406f.

After approximately 50% of the total axial deflection of coupling feature 341 (e.g. of coupling portion 340), it may transition to the angled face, e.g. 412f, on the tooth, e.g. 406f, of number/dial sleeve 400, as shown in figure 7C.

A distance D10c between middle portion 714 of curved face 710 on ramp feature 702 and distally facing segment 349 of curved portion 348 may now be greater than distance D10b. A distance D0c2 between distal face 360 and proximal face 432e of intermediate portion 430e may now be greater than distance D0c.

Edge E2 may have reached and/or may make direct mechanical contact with a point P1c which is located distal compared to point P1b on distal curved portion 346. Edge E1 may have reached and/or may make direct mechanical contact with a point P2c that is more proximal than point P2b. Point P2c may be located on front ramped face 412f.

A minimum distance D11c between edge E2 on ramp feature 702 and distal curved portion 346 of coupling portion 340 may still be zero millimeter due to direct mechanical contact of curved portion 345 and ramped feature 702.

An arrow Arr7Cp illustrates the proximal displacement of coupling feature 341 comprising coupling portion 340. An arrow Arr7Ca illustrates the angular movement of ring member 402.

Figure 7D illustrates a fourth configuration 740 of ring member 402, coupling feature 340 and ramped feature 702. Figure 7D illustrates a cross section through coupling feature 340, showing coupling feature 340 returning to the "left" and riding up the angled face 412f of tooth 404f, more specific of inner portion 406f of tooth 404f.

At this point the spring force that was generated from deflection of the coupling feature 341 and coupling portion 340 tangentially (i.e. to the right or angularly) may be able to return the coupling feature 341 to the left, whereupon the coupling feature 341 can climb up the shallower angled slope of face 412f tooth 404f of number/dial sleeve 400. At the same time, coupling feature 341 may come out of contact with the ramped feature 702 on the button inner 300 (chassis). This is illustrated in figure 7D.

A distance D10d between middle portion 714 of curved face 710 on ramp feature 702 and distally facing segment 349 of curved portion 348 may now be greater than distance D10c. A distance D0d between distal face 360 and proximal face 432e of intermediate portion 430e may now be greater than distance D0c2.

Edge E2 may not make direct mechanical contact with curved portion 345 of front face 343 anymore. Edge E1 may have reached and/or may make direct mechanical contact with a point P2d that is more proximal than point P2c on front ramped face 412f.

A minimum distance D11d between edge E2 on ramp feature 702 and distal curved portion 346 of coupling portion 340 may be greater than distance D11c, e.g. greater than zero millimeter because there is no mechanical contact of curved portion 345 and ramped feature 702 anymore.

An arrow Arr7Dp illustrates the proximal displacement of coupling feature 341 comprising coupling portion 340. An arrow Arr7Da illustrates the angular movement of ring member 402.

Figure 7E illustrates a fifth relative configuration 750 of ring member 402, coupling feature 340 and ramped feature 702. Figure 7E illustrates a cross section through coupling feature 340, showing coupling feature 340 running along the top edge, see point P2e, or top surface of inner portion 406f of tooth 404f of number sleeve 400.

The available 'width' of the teeth, e.g. of the inner teeth, e.g. 406f, of number/dial sleeve 400 may be limited, e.g. by the small lateral or radial dimensions of ring member 402. This means that to achieve the total required axial travel of coupling portion 340 to activate switch Sw, e.g. a micro-switch, or another sensor, based on the angled face on inner teeth alone, the necessary angled face on inner teeth would be too steep and would require too much torque to drive coupling portion 340 up this slope.

By allowing the coupling portion 340 to deflect tangentially (i.e. left to right in the figures 7A to 7E) and adding a slope 710 on ramped feature 702 of button inner 300/chassis, the effective length of this driving slope may be shared between the tooth, e.g. 406f, of number/dial sleeve 400 and the ramped feature 702 on the button inner 300 (chassis), thereby reducing the length/slope of each one individually.

Additionally, the final action of coupling portion 340 may be to rapidly release the energy stored by elastic deformation driving coupling portion 340 back up shallow face 412f on tooth 406f of number/dial sleeve 400, thus minimizing the rotation required from number/dial sleeve 400 to axially displace coupling portion 340 the required distance to activate switch Sw, e.g. the micro-switch, that may turn the e-unit/module (electronic unit/module) 120, 200 on.

The action of axially displacing coupling portion 340 may require additional applied user force to the button inner 300 (chassis), but this may only occur at the start of dispense, before the fluid pressure inside the cartridge may have risen and therefore when the user force to expel fluid is very low. Such an increase to dispense force at the start of dose is still likely to be below a typical force plateau during dose delivery (e.g. at 150 international units I.U. per second) and is therefore not expected to manifest in a noticeable increase in dispense force for the user.

A distance D10e between middle portion 714 of curved face 710 on ramped feature 702 and distally facing segment 349 of curved portion 348 may now be greater than distance D10d. A distance D0e between distal face 360 and proximal face 432e of intermediate portion 430e may now be greater than distance D0d.

Edge E2 may not make direct mechanical contact with curved portion 345 of front face 343. Edge E1 or distal face 362 of back coupling portion 354 may have reached and/or may make direct mechanical contact with a point P2e that is more proximal than point P2c on front ramped face 412f.

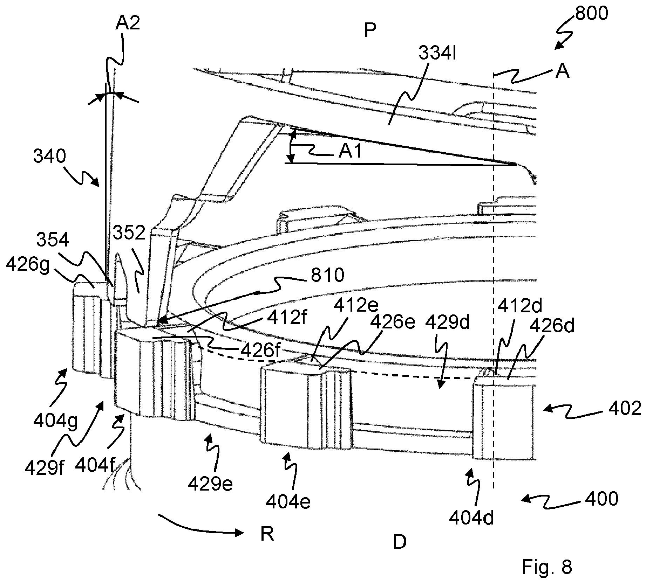

A minimum distance D11e between edge E2 on ramp feature 702 and distal curved portion 346 of coupling portion 340 may be greater than distance D11d. Thus, coupling portion 340 has left inner pocket 427e. As button inner 300 is still pressed, there is a radial deflection of coupling portion 340 that results in radial displacement of coupling portion 340 from inner portion 406f to adjacent inner sub-portion 440f of outer portion 408f of the same tooth 404f. This radial displacement is described in more detail in the following, e.g. with relation to figure 8.

An arrow Arr7Ep illustrates the proximal displacement of coupling feature 341 comprising coupling portion 340. An arrow Arr7Ea illustrates the angular movement of ring member 402.

The same is valid if the coupling feature 340 is straddling over an inner teeth, e.g. 406f. In this case, the rotation force is impacted to the front coupling portion 352 of coupling portion 340. Again, ramped feature 702 moves coupling feature 341 and therefore also coupling portion 340 proximally by sliding along cured surface 345. As soon as ramped face, e.g. 412f, of tooth, e.g. 406f, engages face 360, e.g. the edge formed between distal face 360 and back face 353, the proximal displacement of coupling feature 340 may be caused completely or mainly by the ramped face, e.g. 412f, e.g. the supporting feature 702 may have no contact to the coupling feature 340 anymore.

Figure 8 illustrates the coupling feature 340 in a deflected state, e.g. deflected upwards and deflected radially outwards. For the driving force to remain low for the duration of the drug dispense event, it may be important that the coupling portion 340 (comb) continues to run on the flat top face or faces 426d, 426e, 426f, 426g, etc. of the teeth 404d, 404e, 404f and 404g of the number/dial sleeve 400 and do not drop back down into outer pockets 429d, 429e and 429f between teeth 404e, 404f and 404g of the number/dial sleeve 400. Teeth 404e, 404f and 404g may be part of a clutch of the drug delivery device 100.

This may be achieved by making use of the fact that as the coupling portion 340 (comb) deflects axially upwards it may deflect also radially outwards, especially at the distal tip, e.g. as shown in figure 8, arrow 810. For the illustrated design, a finite element analysis may have predicted that for 0.6 mm (millimeter) axial deflection, the tip may deflect 0.42 mm radially outwards. This radial outwards displacement may happen as the coupling portion 340 (comb) climbs out of the inner pocket 327e at the end of angled face 412d, 412e, 412f, etc. of one of the inner teeth (inner portion) 406d, 406e, 406f, etc. and may allow the coupling portion 340 to run along the flat faces 426d, 426f, 426g, etc. on the top of the teeth 404d, 404f, 404g, etc. of the number/dial sleeve 400, especially on the flat surfaces of inner sub-portions 442f, 442g, etc.

Configuration 800 illustrates coupling portion 340 on top of teeth 404f and 404g. There may be an angle A1 between the left portion 334l of ring 330, especially between the lower side thereof, in the axially (proximally) most deflected position, and a reference plane that is parallel to a plane in which coupling ring 402 is arranged. The reference plane may correspond to the plane in which the lower side of the left portion 334l of ring 330 is arranged in its released position, e.g. in the position where no axial deflection of left portion 334l occurs. Angle A1 may be in the range of 1 to 5 degrees or in the range of 2 to 4 degrees or within another appropriate range.

An angle A2 may be formed between the outside of coupling portion 340 or of the whole coupling feature 341 and an axis that is parallel to longitudinal axis A. Angle A2 may have the same value as angle A1. Alternatively, angle A2 may be less, e.g. if coupling portion 340 is inclined slightly inwards in its released state. According to a further alternative, angle A2 may be greater than angle A1.

Left portion 334l of holding ring 330 and/or right portion of holding ring 330 may be straight or may have a different form, e.g. cranked.

Figures 9A and 9B illustrate how the coupling feature 340 is configured to bridge the inner sub-portions 440f and outer pockets 429e (gaps) between teeth, e.g. teeth 404e and 404f, of number sleeve 400 to continue to run on flat faces 426d, 426f, 426g, etc.

A configuration 900 is illustrated in figure 9A. The following equation may be valid:

$$W3a > W1a, (3),$$

wherein W3a is the angular width of an inner sub-portion, e.g. 440f, of the outer portion(s), e.g. 408f, and
wherein W1a is the angular width of the recessed portion 350 of the coupling portion 340.

Angular width W3a may be measured from leading edge 444f or inner sub-portion 440f of a respective tooth 404f to the back face of the same respective tooth 404f, e.g. the back face 460f of the inner sub-portion 440f of a respective tooth 404f, see figure 9, corresponding back face 460e of tooth 404e. Angular width W1a of recessed portion 350 may be measured from back face of front coupling portion 352 to front face 355 of back coupling portion 354. Thus, the distal portion 340c of coupling portion 340 is supported by the inner sub-portion, e.g. 440f, etc. if above one of the teeth, e.g. 404f, etc. Moreover, recess 350 may not engage with inner portions 406f in the radial outwards position of coupling portion 340.

A configuration 910 is illustrated in figure 9B. The following equation may be valid:

$$W1b > D3b, (4),$$

wherein W1b is the angular width of the coupling portion 340, and
wherein D3b is the angular distance between a back face, e.g. 460e, of an inner sub-portion, e.g. 440e, of the outer portion, e.g. 408e, and a front face or a front edge, e.g. 444f, of the adjacent inner sub-portion, e.g. 444f, of the adjacent outer portion, e.g. 408f.

Angular width W1b may be measured between the vertical portion of front face 344 (e.g. at the distal end) and the vertical back face 342 of coupling portion 340.

Thus, the distal portion 340c of coupling feature 340 may not fall back into the intermediate space or into outer pocket 429e between two adjacent inner sub-portions 440e, 440f of outer portions 408e since at least one of the front coupling portion 252 or of the back coupling portion 354 is supported by an inner sub-portion 440e, 440f, etc. Outer portions 408e and 408f are illustrated in figure 9B.

Alternatively, a closed circumferential ring may be used on inner teeth 406a to 406f instead of inner sub-portions 440e, 440f, etc., e.g. there may be intermediate portions connecting inner sub-portion 440e, 440f, etc. in circumferential or angular direction.

According to another embodiment an outer holding ring of number sleeve 400 may be used instead of the inner sub-portion 440e, 440f, etc. thereby omitting the outer sub-portions 442e, 442f, etc., e.g. no outer portions 408e and 408f may be used for this modification. The outer ring may not be attached directly to inner teeth 406a to 406f, e.g. there may be a radial gap between inner teeth 406a to 406f and the outer holding ring. However, as outer holding ring may also be arranged onto ring member 402 it rotates in common with inner teeth 406a to 406f. Coupling portion 340 slides on the distal face of outer holding ring in this variant.

Figure 9B illustrates a back face 460e of inner sub-portion 440e as well as a back face 462e of outer sub-portion 442e of outer portion 408e. As illustrated, back face 460e and back face 462e may be arranged within the same vertical plane in the embodiment shown. Alternatively, other arrangements of these two back faces 260e and 262e may also be used.

The following equation may be valid:

$$W3a > W3b, (5),$$

wherein W3a is the angular width of an inner sub-portion, e.g. 440e, of the outer portion(s), e.g. 408e, and
W3b is the angular width of an outer sub-portion, e.g. 442e, of the outer portion(s), e.g. 408e.

Angular width W3a may be measured as explained above for equation (3). However, reference may be made to tooth 404e although it is assumed that all teeth 404e, 404f have identical shapes and specific dimensions. Angular width W3b of outer sub-portion 442e may be measured from front face 480e of outer sub-portion 442e to back face 462e of outer sub-portion 442e. Thus, outer sub-portion 442e may be adapted to have further functions if compared to the function of inner sub-portion(s) 440e, etc. and/or compared to the function of inner portions(s) 406e, etc. Exemplary, outer sub-portion 442e may have a further coupling function by forming clutch teeth of a clutch of drug delivery device 100.

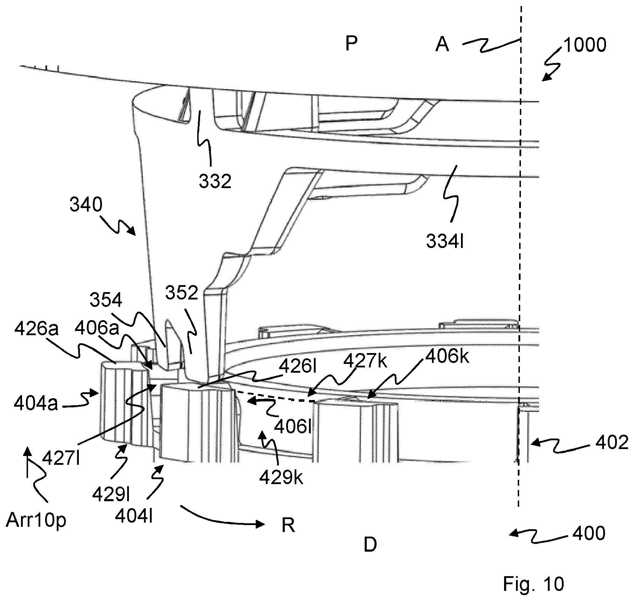

Figure 10 illustrates a position of coupling feature 340 in which it is positioned again above the inner pockets between the teeth, e.g. above inner pocket(s) 427l between inner portions 406l and 406a and not on top faces 426a and 426l anymore, see configuration 1000.

At the end of a dispense event when the user releases the button, button inner 300 may move axially away from the top face of number/dial sleeve 400 and as it does so, the flexible switcher arms, e.g. left portion 334l of ring 330 and right portion 334r of ring 330 may relax back to their original state with the tip of coupling portion 340 (comb) on the smaller radius whereupon it can drop back down into inner pocket(s) 427l, etc. between inner portions, e.g. 406k and 406l, or into engagement with one of these inner portions, e.g. 406k and 406l, e.g. depending on the rotational end position of coupling ring 402 after dose delivery.

In this condition, if the user depresses the button inner 300 again without number/dial sleeve 400 being allowed to rotate (i.e. not during dose delivery) then the coupling portion 340 (comb) may be positioned radially over the ramped inner pockets 427k, 427l, etc. rather than over the flat surfaces/faces 426l, 426a. Therefore, coupling portion 340 will be not deflected axially and/or radially and switch Sw will not be activated during this depressing of button inner 300, see figure 10. An arrow Arr10d indicates the proximal movement of button inner 300 and of coupling portion 340 in order to release coupling portion 340 from the flat faces 426l, 426a, etc.

The angle A1 mentioned above will be again zero degrees after release of the button, e.g. after release of the button inner 300. Again, left portion 334l of ring 330 and/or right portion of ring 330 may be straight or may have a different form, e.g. cranked.

One aspect of the disclosure described within this application may be the method of waking a clip-on electronic module/unit 120, 200 using rotation of the mechanism, designed for use in conjunction with an injection device 100.

The benefit to waking the electronic module 120, 200 on or shortly before the beginning of dose delivery may be that power consumption of the dose capture system can be limited by having the capture system active for the shortest possible time.

This may be particularly useful for systems such as an optical encoder, where the power consumption of the IR-LED (infrared - light emitting diode) sensors accounts for a significant proportion of the total power consumption of the electronic module 120, 200.

Thus, an actuating unit 301 for a drug delivery device 100, may comprise:

- A support structure 300 extending along a longitudinal axis A between a distal D portion of the support structure 300 and a proximal P portion of the support structure 300, and/or
- An interface unit IF1, IF2 arranged within the support structure 300 and comprising a coupling portion 340 and an actuating portion 332,

wherein the coupling portion 340 is configured to interact with a rotating member comprising a plurality of teeth 404a and rotating around a rotation axis A that is parallel or coaxial to the longitudinal axis A,
wherein the actuating portion 332 is configured to interact with an electrical component Sw, wherein the interface unit IF1, IF2 is configured to interact with the plurality of teeth 404a of the rotating member and to convert a rotational movement R of the rotating member to an axial displacement Arr7Bp of the coupling portion 340 in a first direction relative to the longitudinal axis A, e.g. from a first axial position to a second axial position, and to a radial displacement of at least a part of the coupling portion 340 relative to the longitudinal axis,
wherein the axial displacement and/or the radial displacement of the coupling portion 340 displaces the actuating portion 332, and
wherein the direction and amount of the radial displacement is appropriate to hold the coupling portion 340 with a holding structure 440f arranged, e.g. on the rotating member, thereby preventing re-engagement of the coupling portion 340 with the teeth 404a of the rotating member.

Furthermore, a rotating member may comprise:

- A ring member 330, and
- A plurality of teeth 404a arranged circumferentially on the ring member 330 around an axis A of rotation of the ring member 330,

each tooth 404a may comprise an inner portion 406a and an outer portion 408a arranged radially outwards for the respective inner portion 406a,
wherein the inner portions 406a comprise first teeth comprising at least one ramped face 412a that faces in a proximal direction relative to the axis A of rotation and in circumferential direction of the ring member 330, and wherein the outer portions 408a comprise second teeth of a different orientation and/or type compared to the first teeth, wherein the second teeth comprise a flat face 426d that is arranged in a plane perpendicular to the axis A of rotation and wherein the second teeth have an angular width W3a, W3b that is greater than an angular width W2a of the first teeth, and
wherein a most proximal portion P2e of the first teeth extends up to the plane.

A number sleeve 400 may be claimed comprising the actuating unit 301 and the rotating member.

A drug delivery device 100 is disclosed, e.g. pen type device, comprising the actuating member 301 and/or the rotating member, e.g. ring member 330.

Moreover, a method for actuating a drug delivery device 100 may comprise:

- Pressing an actuating unit 301 distally thereby engaging a coupling portion 340 and teeth 404 of a rotating member,
- Thereafter, rotating R the rotating member thereby disengaging the coupling portion 340 out of a pocket formed between adjacent teeth 404a, 404b, whereby an actuating portion 332 of the actuating unit 301 acts on the electrical component Sw, and
- Thereafter, preventing the coupling portion 340 from re-engaging pockets between the teeth 404a, 404b, whereby preferably the actuating portion 332 of the actuating unit 301 acts further on the electrical component Sw.

A system 98 is disclosed, comprising:

- A drug delivery device 100, and
- An electronic module 120, 200,

wherein the drug delivery device 100 comprises a rotating member comprising a plurality of teeth 404a,
wherein the electronic module 120, 200 comprises an electrical component Sw, an electrical energy source Bat and an energy management unit electrically connected to the at least one electrical component Sw,
wherein the energy management unit is configured to switch depending on an electrical signal generated by the electrical component Sw from a low energy state to a higher energy state that consumes more electrical energy from the electrical energy source Bat compared to the low energy state,
wherein the electronic module comprises an interface unit IF1, IF2,
wherein the interface unit IF1, IF2 comprises a coupling portion 340 and an actuating portion 332 configured to interact with the electrical component Sw,
wherein the interface unit IF1, IF2 is configured to interact with the plurality of teeth 404a of the rotating member by the coupling portion 340 and to convert a rotational movement R of the rotating member to an axial displacement Arr7Bp of the coupling portion 340 in a first axial direction relative to a longitudinal axis A of the drug delivery device 100 and to a radial displacement of at least a part of the coupling portion 340 relative to the longitudinal axis A,
wherein the interface unit IF1, IF2 is configured to transfer the axial displacement Arr7Bp and/or the radial displacement 810 to the actuating portion 332 thereby initiating switching to the higher energy state by the energy management unit, and
wherein the direction and amount of the radial displacement is appropriate to hold the coupling portion 340 by a holding structure 440f thereby preventing re-engagement of the coupling portion 340 and the teeth 404a of the rotating member during dispense of a dose.

Although embodiments of the present disclosure and their advantages have been described in detail, it should be understood that various changes, substitutions and alterations can be made therein without departing from the spirit and scope of the disclosure as defined by the appended claims. For example, it will be readily understood by those skilled in the art that many of the features, functions, processes and methods described herein may be varied while remaining within the scope of the present disclosure. Moreover, the scope of the present application is not intended to be limited to the particular embodiments of the system, process, manufacture, method or steps described in the present disclosure. As one of ordinary skill in the art will readily appreciate from the disclosure of the present disclosure, systems, processes, manufacture, methods or steps presently existing or to be developed later that perform substantially the same function or achieve substantially the same result as the corresponding embodiments described herein may be utilized according to the present disclosure. Accordingly, the appended claims are intended to include within their scope such systems, processes, methods or steps. Further, it is possible to combine embodiments mentioned in the first part of the description with examples of the second part of the description which relates to Figures 1 to 10.

**Claims**

**1.** Actuating unit (301) for a drug delivery device (100), comprising:

a support structure (300) extending along a longitudinal axis (A) between a distal (D) portion of the support structure (300) and a proximal (P) portion of the support structure (300), and
an interface unit (IF1, IF2) arranged within the support structure (300) and comprising a coupling portion (340) and an actuating portion (332),
wherein the coupling portion (340) is configured to interact with a rotating member comprising a plurality of teeth (404a) and rotating around a rotation axis (A) that is parallel or coaxial to the longitudinal axis (A),
wherein the actuating portion (332) is configured to interact with an electrical component (Sw),
wherein the interface unit (IF1, IF2) is configured to interact with the plurality of teeth (404a) of the rotating member and to convert a rotational movement (R) of the rotating member to an axial displacement (Arr7Bp) of the coupling portion (340) relative to the longitudinal axis (A) and
to a radial displacement of at least a part of the coupling portion (340) relative to the longitudinal axis (A), and
wherein the axial displacement and/or the radial displacement of the coupling portion (340) displaces the actuating portion (332).

**2.** Actuating unit (301) according to claim 1, wherein the direction and amount of the radial displacement is appropriate to hold the coupling portion (340) with a holding structure (440f) thereby preventing re-engagement of the coupling portion (340) with the teeth (404a) of the rotating member during dispense of a dose.

**3.** Actuating unit (301) according to claim 1 or 2, wherein the interface unit (IF1, IF2) comprises at least one retaining element (3341, 334r) that retains the coupling portion (340) on a retaining portion of the retaining element (334l, 334r),

wherein the retaining element (334l, 334r) is mounted to the supporting structure (300) with an attachment portion of the retaining element (334l, 334r),
wherein the retaining element (334l, 334r) is resilient and/or is attached pivotable to the supporting structure (300) thereby allowing the distal displacement and the radial displacement of the coupling portion (340).

**4.** Actuating unit (301) according to any one of the claim 1 to 3, wherein the coupling portion (340) is configured to interact with an interacting portion on a respective one of the teeth (404a) of the rotating member such that the rotating member acts a force (F1) on the coupling portion (340),

wherein the rotating member comprises at least one holding structure (440f) arranged radially outwards of the interaction portion of the rotating member,
wherein the holding structure (440) comprises at least one proximal directed surface (426d) configured to allow sliding of the distal end (360, 362) of the coupling portion (340) thereon during further rotation of the rotating member.

**4.** Actuating unit (301) according to any of the previous claims, comprising a coupling feature (341) wherein the coupling portion (340) is comprised within the coupling feature (341), wherein the coupling feature (341) comprises at least one curved face (345) configured to interact with a guiding feature (702) mounted to the supporting structure (330) when a circumferential force (F1) is applied by the rotating member to the coupling portion (340) thereby displacing the coupling feature (341) axially,

and wherein preferably the guiding feature (702) comprises at least one curved face (710) opposite to the curved face (345) of the coupling feature (341),
wherein preferably both curved surfaces (710, 345) are formed complementary to each other thereby promoting the axial displacement of the coupling feature (341).

**6.** Actuating unit (301) according to any of the previous claims, wherein the coupling portion (340) comprises at least two distal portions (352, 354) and a recessed portion (350) arranged between the at least two distal portions (352, 354),
wherein the recessed portion (350) is recessed proximally relative to the at least two distal portions (352, 354).

**7.** Actuating unit (301) according to claim 6, wherein a first distal portion (352) of the at least two distal portions (352, 354) comprises a first side face (353) of the recessed portion (350) and a second distal portion (354) of the at least two distal portions (352, 354) comprises a second side face (355) of the recessed portion (350) and wherein the second side face (355) is longer than the first side face (353).

**8.** Rotating member, comprising:

a ring member (402),
a plurality of teeth (404a) arranged circumferentially on the ring member (402) around an axis (A) of rotation of the ring member (402),
each tooth (404a) comprising an inner portion (406a, 406k) and an outer portion (408a, 408k) arranged radially outwards for the respective inner portion (406a, 406k),
wherein the inner portions (406a, 406k) comprise first teeth comprising at least one ramped face (412a, 412d) that faces in a proximal direction relative to the axis (A) of rotation and in circumferential direction of the ring member (402), and
wherein the outer portions (408a, 408k) comprise second teeth of a different orientation and/or type compared to the first teeth,
wherein the second teeth comprise a flat face (426d) that is arranged in a plane perpendicular to the axis (A) of rotation and wherein the second teeth have an angular width (W3a, W3b) that is greater than an angular width (W2a) of the first teeth, and
wherein a most proximal portion (P2e) of the first teeth or a ramp starting at this proximal portion (P2e) extends up to the plane.

**9.** Rotating member, according to claim 8, wherein the outer portions (408a, 408k) comprise respectively an inner sub-portion (440f) and an outer sub-portion (442f) arranged radially outwards of the inner sub-portion (440f),
wherein the inner sub-portions (440f) have an angular width (W3a) that is larger than the angular width (W3b) of the outer sub-portion.

**10.** Drug delivery device (100), comprising:

a housing (102) extending along a longitudinal axis (A),
a dose setting unit and/or a drug delivery unit arranged within the housing (102),
an actuating unit (301) according to one of the claims 1 to 7,
wherein the actuating unit (301) is arranged on or within the housing (102) and wherein the actuating unit (301) comprises an electric component (Sw) that is actuated by the actuating portion (332) of the actuating unit (301), and/or the rotating member according to claim 8 or 9,
wherein the rotating member is part of or is mechanically coupled to the dose setting unit and/or to the dose delivery unit,
and wherein the rotating member is arranged such that it interacts with the actuating unit (301) or wherein the rotating member is configured to interact with the actuating unit (301).

**11.** Drug delivery device (100) according to claim 10, wherein the dose setting unit and/or the dose delivery unit is configured to position the rotating member in one of a number of positions, and
wherein the number of teeth (404a) of the rotating member is equal to one half of the number of positions.

**12.** Drug delivery device (100) according to claim 10 or 11, wherein at least one, at least two, at least three or all of the following equations are valid:

$$W1a > W2a, \quad (1),$$

wherein $W1a$ is the angular width of a recessed portion (350) or of the recessed portion (350) of the coupling portion (340), and
wherein $W2a$ is the angular width of an inner tooth (406a) or of the inner tooth (404a) of the rotating member,

$$D2b > W1b, \quad (2),$$

wherein $D2b$ is the angular distance between a back face (416e) of a first one of the inner tooth (404e) and the front face (410f) an adjacent inner tooth (404f), and

wherein W1b is the angular width of the coupling portion (340),

$$W3a > W1a, (3),$$

wherein W3a is the angular width of an inner sub-portion (440f) of the outer portions (408f), and
wherein W1a is the angular width of a recessed portion (350) or of the recessed portion (350) of the coupling portion (340),

$$W1b > D3b, (4),$$

wherein W1b is the angular width of the coupling portion (340), and
wherein D3b is the angular distance between a back face (460e) of an inner sub-portion (440e) of the outer portion (408e) and a front face or a front edge (444f) of the adjacent inner sub-portion (444f) of the adjacent outer portion (408f).

13. Drug delivery device (100) according to any of the claims 10 to 12, wherein the coupling portion (340) is configured to interact with an interacting portion (406e) on a respective one of the teeth (404a) of the rotating member such that the rotating member acts a force (F1) on the coupling portion (340),

wherein the rotating member comprises at least one holding structure (440e, 470e) arranged radially outwards of the interaction portion (406e) of the rotating member,
wherein the holding structure (440e, 470e) comprises at least one proximal directed face (470e) configured to allow sliding of the distal end (360, 362) of the coupling portion (340) thereon during further rotation of the rotating member.

14. Drug delivery device (100) according to any of the claims 10 to 13, wherein the following equation is valid:

$$W3a > W3b, (5),$$

wherein W3a is the angular width of an inner sub-portion (440f) of the outer portions (408f), and W3b is the angular width of an outer sub-portion (442e) of the outer portions (408e).

15. Method for actuating a drug delivery device (100), comprising:

pressing an actuating unit (301) distally thereby engaging a coupling portion (340) and teeth (404) of a rotating member,
thereafter, rotating (R) the rotating member thereby disengaging the coupling portion (340) out of a pocket formed between adjacent teeth (404a, 404b), whereby an actuating portion (332) of the actuating unit (301) acts on the electrical component (Sw), and
thereafter, preventing the coupling portion (340) from re-engaging pockets between the teeth (404a, 404b), whereby preferably the actuating portion (332) of the actuating unit (301) acts further on the electrical component (Sw).

16. System (98), comprising:

a drug delivery device (100), and
an electronic module (120, 200),
wherein the drug delivery device (100) comprises a rotating member comprising a plurality of teeth (404a),
wherein the electronic module (120, 200) comprises an electrical component (Sw), an electrical energy source (Bat) and an energy management unit electrically connected to the at least one electrical component (Sw),
wherein the energy management unit is configured to switch depending on an electrical signal generated by the electrical component (Sw) from a low energy state to a higher energy state that consumes more electrical energy from the electrical energy source (Bat) compared to the low energy state,
wherein the electronic module comprises an interface unit (IF1, IF2),
wherein the interface unit (IF1, IF2) comprises a coupling portion (340) and an actuating portion (332) configured to interact with the electrical component (Sw),
wherein the interface unit (IF1, IF2) is configured to interact with the plurality of teeth (404a) of the rotating member by the coupling portion (340) and to convert a rotational movement (R) of the rotating member to an axial displacement (Arr7Bp) of the coupling portion (340) in a first axial direction relative to a longitudinal axis (A) of the drug delivery device (100) and to a radial displacement of at least a part of the coupling portion (340) relative to the longitudinal axis (A),
wherein the interface unit (IF1, IF2) is configured to transfer the axial displacement (Arr7Bp) and/or the radial displacement (810) to the actuating portion (332) thereby initiating switching to the higher energy state by the energy management unit, and
wherein the direction and amount of the radial displacement is appropriate to hold the coupling portion (340) by a holding structure (440f) thereby preventing re-engagement of the coupling portion (340) and the teeth (404a) of the rotating member during dispense of a dose.

17. System (98) according to claim 16, wherein the interface unit (IF1, IF2) is configured to prevent interaction of the actuating portion (332) and of the electrical component (Sw) when the coupling portion (340) is moved in a second axial direction that is opposite to the first axial direction.

Fig. 1

Fig. 2

Fig. 3

Fig. 4

Fig. 5A

Fig. 5B

Fig. 6A

Fig. 6B

Fig. 7A

Fig. 7B

Fig. 7C

Fig. 7D

Fig. 7E

Fig. 8

Fig. 9A

Fig. 9B

Fig. 10

Europäisches
Patentamt

European
Patent Office

Office européen
des brevets

# EUROPEAN SEARCH REPORT

Application Number

EP 21 31 5262

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/191329 A1 (SANOFI SA [FR]) 30 September 2021 (2021-09-30) | 8,9 | INV. A61M5/20 A61M5/315 A61M5/24 |
| A | * page 21, line 14 – page 34, line 20 * * figures 1-6 * | 1-7, 10-17 | |
| A | US 2021/170111 A1 (COLLINGS RALPH DONALD QUENTIN [GB] ET AL) 10 June 2021 (2021-06-10) * paragraphs [0107] – [0162] * * figures 1-34 * | 1-17 | |

| | |
|---|---|
| **TECHNICAL FIELDS SEARCHED** (IPC) | |
| A61M | |

The present search report has been drawn up for all claims

| Place of search | Date of completion of the search | Examiner |
|---|---|---|
| Munich | 12 September 2022 | Pisseloup, Arnaud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 31 5262

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2022

| Patent document cited in search report | | Publication date | Patent family member(s) | | Publication date |
|---|---|---|---|---|---|
| WO 2021191329 | A1 | 30-09-2021 | NONE | | |
| US 2021170111 | A1 | 10-06-2021 | CN | 111511421 A | 07-08-2020 |
| | | | EP | 3727509 A1 | 28-10-2020 |
| | | | JP | 2021507749 A | 25-02-2021 |
| | | | US | 2021170111 A1 | 10-06-2021 |
| | | | WO | 2019122085 A1 | 27-06-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014033195 A1
- WO 2014033197 A1
- WO 2005018721 A
- US 6004297 A
- US 9108002 B